(19) Europäisches Patentamt
European Patent Office
Office européen des brevets

(11) **EP 4 623 827 A1**

(12) **EUROPEAN PATENT APPLICATION**
published in accordance with Art. 153(4) EPC

(43) Date of publication:
**01.10.2025 Bulletin 2025/40**

(21) Application number: **23918489.8**

(22) Date of filing: **14.09.2023**

(51) International Patent Classification (IPC):
***A61B 6/00*** (2024.01)

(52) Cooperative Patent Classification (CPC):
**A61B 6/4225; A61B 6/00; A61B 6/5258**

(86) International application number:
**PCT/JP2023/033577**

(87) International publication number:
**WO 2024/157532 (02.08.2024 Gazette 2024/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.01.2023 JP 2023008185**

(71) Applicant: **Hamamatsu Photonics K.K.
Hamamatsu-shi, Shizuoka 435-8558 (JP)**

(72) Inventors:
• **TSUCHIYA, Satoshi
Hamamatsu-shi, Shizuoka 435-8558 (JP)**
• **ONISHI, Tatsuya
Hamamatsu-shi, Shizuoka 435-8558 (JP)**

(74) Representative: **Hoffmann Eitle
Patent- und Rechtsanwälte PartmbB
Arabellastraße 30
81925 München (DE)**

(54) **IMAGE PROCESSING METHOD, TRAINING METHOD, TRAINED MODEL, RADIOLOGICAL IMAGE PROCESSING MODULE, RADIOLOGICAL IMAGE PROCESSING PROGRAM, AND RADIOLOGICAL IMAGE PROCESSING SYSTEM**

(57)     An image processing method includes an image acquisition step of acquiring an image obtained by irradiating a subject F with an energy beam and capturing an image of the energy beam transmitted through the subject F, a spatial blur map generation step of generating a spatial blur map indicating a distribution of spatial blur of noise based on the image, and a processing step of inputting the image and the spatial blur map into a trained model 207 constructed in advance through machine learning and executing image processing for removing noise from the image.

Fig.11

EP 4 623 827 A1

## Description

### Technical Field

One aspect of an embodiment relates to an image processing method, a training method, a trained model, a radiological image processing module, a radiological image processing program, and a radiological image processing system.

### Background Art

Since the past, methods of removing noise from an image using machine learning have been known. For example, Patent Literature 1 discloses an image processing method of removing noise from a radiological image. This image processing method includes deriving an evaluation value obtained by evaluating a spread of noise from a pixel value of each pixel in a radiological image based on relationship data indicating a relationship between the pixel value (luminance value) and the evaluation value, generating a noise map that is data in which the derived evaluation value is associated with each pixel in the radiological image, and inputting the noise map and the radiological image into a trained model.

### Citation List

### Patent Literature

[Patent Literature 1] International Patent Publication WO 2022/172506

## Summary of Invention

### Technical Problem

In the image processing method as described above, noise in an image is removed in consideration of the spread of noise in a luminance direction. For example, noise in an image is removed in consideration of the relationship between the luminance value and the standard deviation of pixel values. However, in a case where the blur of noise which is the spread of noise in a spatial direction changes in the image, a sufficient noise removal effect may not be obtained.

Consequently, one aspect of an embodiment was contrived in view of such a problem, and an subject thereof is to provide an image processing method, a training method, a trained model, a radiological image processing module, a radiological image processing program, and a radiological image processing system that make it possible to cope with a change in the blur of noise in an image.

### Solution to Problem

According to one aspect of an embodiment, there is provided an image processing method including: an image acquisition step of acquiring an image obtained by irradiating a subject with an energy beam and capturing an image of the energy beam transmitted through the subject; a spatial blur map generation step of generating a spatial blur map indicating a distribution of spatial blur of noise based on the image; and a processing step of inputting the image and the spatial blur map into a trained model constructed in advance through machine learning and executing image processing for removing noise from the image.

Alternatively, according to another aspect of the embodiment, there is provided a radiological image processing module comprising: an image acquisition unit configured to acquire a radiological image obtained by irradiating a subject with radiation and capturing an image of the radiation transmitted through the subject; a spatial blur map generation unit configured to generate a spatial blur map indicating a distribution of spatial blur of noise based on the radiological image; and a processing unit configured to input the radiological image and the spatial blur map into a trained model constructed in advance through machine learning and execute image processing for removing noise from the radiological image.

Alternatively, according to another aspect of the embodiment, there is provided a radiological image processing program causing a processor to function as: an image acquisition unit configured to acquire a radiological image obtained by irradiating a subject with radiation and capturing an image of the radiation transmitted through the subject; a spatial blur map generation unit configured to generate a spatial blur map indicating a distribution of spatial blur of noise based on the radiological image; and a processing unit configured to input the radiological image and the spatial blur map into a trained model constructed in advance through machine learning and execute image processing for removing noise from the radiological image.

Alternatively, according to another aspect of embodiment, there is provided an radiological image processing system including: the radiological image processing module; a source configured to irradiate the subject with radiation; and an imaging device configured to capture an image of the radiation transmitted through the subject to acquire the radiological image.

**Advantageous Effects of Invention**

According to one aspect the present invention, it is possible to provide an image processing method, a training method, a trained model, a radiological image processing module, a radiological image processing program, and a radiological image processing system that make it possible to cope with a change in the blur of noise in an image.

**Brief Description of Drawings**

FIG. 1 is a schematic configuration diagram of an image acquisition device 1 according to an embodiment.

FIG. 2 is a block diagram illustrating an example of a hardware configuration of a control device 20 in FIG. 1.

FIG. 3 is a block diagram illustrating a functional configuration of the control device 20 in FIG. 1.

FIG. 4 is a diagram illustrating an example of an X-ray image acquired by an image acquisition unit 201 in FIG. 3.

FIG. 5 is a diagram illustrating an example of generation of a noise map which is performed by a noise map generation unit 202 in FIG. 3.

FIG. 6 is a diagram illustrating an example of generation of a spatial blur map which is performed by a spatial blur map generation unit 203 in FIG. 3.

FIG. 7 is a diagram illustrating spatial blur evaluated based on blur evaluation information in the spatial blur map of FIG. 6.

FIG. 8 is a diagram illustrating a method of deriving second relationship data G70 indicating the correspondence relation between a pixel value and a sigma value in FIG. 6.

FIG. 9 is a graph illustrating a relationship referenced when the second relationship data G70 indicating the correspondence relation between the pixel value and the sigma value in FIG. 6 is derived.

FIG. 10 is a diagram illustrating a method of deriving the second relationship data G70 indicating the correspondence relation between the pixel value and the sigma value in FIG. 6.

FIG. 11 is a diagram illustrating an example of input and output data of a trained model 207 in FIG. 3.

FIG. 12 is a diagram illustrating an example of generation of training data which is performed by a training data generation unit 206 in FIG. 3.

FIG. 13 is a flowchart illustrating a procedure of observation processing performed by the image acquisition device 1.

FIG. 14 is a diagram illustrating an example of X-ray images before and after noise removal processing acquired by the image acquisition device 1.

**Description of Embodiments**

Hereinafter, an embodiment of the present invention will be described in detail with reference to the accompanying drawings. Meanwhile, in the description, the same elements or elements having the same function are denoted by the same reference signs, and thus duplicate description will be omitted.

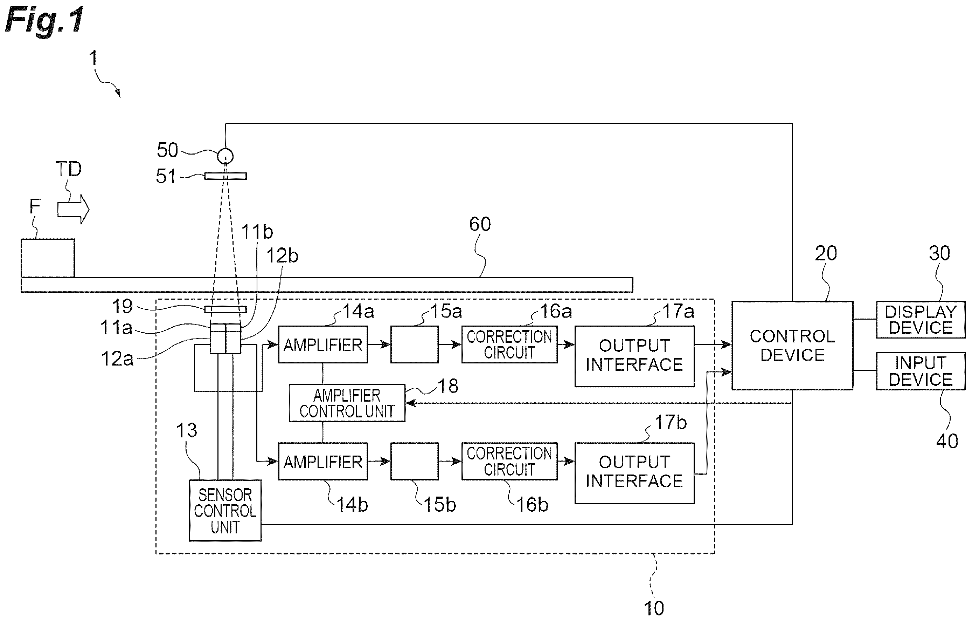

FIG. 1 is a configuration diagram of an image acquisition device 1 which is a radiological image processing system according to an embodiment. As shown in FIG. 1, the image acquisition device 1 is a device that irradiates a subject F transported in a transport direction TD with X-rays (energy beam) (radiation) and acquires an X-ray image (image) (radiological image) obtained by capturing an image of the subject F based on the X-rays transmitted through the subject F. The image acquisition device 1 performs a foreign substance inspection, a weight inspection, a product inspection, or the like on the subject F using an X-ray image, and examples of the application include a food inspection, a baggage inspection, a substrate inspection, a battery inspection, a material inspection, and the like. The image acquisition device 1 is configured to include a belt conveyor (transport means) 60, an X-ray irradiator (source) 50, an X-ray detection camera (imaging device) 10, a control device (radiological image processing module) 20, a display device 30, and an input device 40 for performing various inputs. Meanwhile, in the embodiment of the present disclosure, the image need only be an image obtained by capturing an image of an energy beam transmitted through the subject F. The energy beam may be, for example, radiation such as X-rays and γ-rays, or may be any of visible light, infrared rays, ultraviolet rays, and electron beams. In the present embodiment, the image is a radiological image such as an X-ray image, but may be any other image.

The belt conveyor 60 has a belt portion on which the subject F is placed, and transports the subject F in the transport direction TD at a predetermined transport speed by moving the belt portion in the transport direction TD. The transport speed of the subject F is, for example, 48 m/min. The belt conveyor 60 can change the transport speed as necessary to a transport speed such as, for example, 24 m/min or 96 m/min. In addition, the belt conveyor 60 can appropriately change the height position of the belt portion to change a distance between the X-ray irradiator 50 and the subject F. Meanwhile, examples of the subject F transported by the belt conveyor 60 include foodstuffs such as meat, seafood, agricultural products, or confectionery, rubber products such as tires, resin products, metal products, resource materials such as minerals, waste, and various products such as electronic parts or electronic substrates. The X-ray irradiator 50 is a device that radiates (outputs) X-rays to the subject F as an X-ray source. The X-ray irradiator 50 is a point light source, and diffuses and radiates the X-rays in a predetermined angle range in a fixed irradiation direction. The X-ray irradiator 50 is disposed above the belt conveyor 60 at a predetermined distance from the belt conveyor 60 so that the irradiation direction of the X-rays is directed toward the belt conveyor 60 and the diffused X-rays extend in the entire width direction of the subject F (a direction intersecting the transport direction TD). In addition, in the lengthwise direction of the subject F (a direction parallel to the transport direction TD), the irradiation range of the X-ray irradiator 50 is set as a predetermined division range in the lengthwise direction, and the X-rays are radiated in the entire lengthwise direction of the subject F by the subject F being transported in the transport direction TD by the belt conveyor 60. The tube voltage and tube current of the X-ray irradiator 50 are set by the control device 20. The X-ray irradiator 50 irradiates the belt conveyor 60 with X-rays having predetermined energy and a predetermined radiation dose according to the set tube voltage and tube current. In addition, a filter 51 that transmits a predetermined wavelength region of the X-rays is provided in the vicinity of the X-ray irradiator 50 on the belt conveyor 60 side. The filter 51 is not necessarily required and may be omitted as appropriate.

The X-ray detection camera 10 detects X-rays passing through the subject F among the X-rays radiated to the subject F by the X-ray irradiator 50, and outputs a signal based on the X-rays. The X-ray detection camera 10 is a dual-line X-ray camera in which two sets of configurations for detecting X-rays are disposed. In the image acquisition device 1 according to the present embodiment, each X-ray image is generated based on the X-rays detected in each line (a first line and a second line) of the dual-line X-ray camera. By performing average processing, addition processing, or the like on the two generated X-ray images, a clear (high-luminance) image can be acquired with a smaller X-ray dose than in a case where an X-ray image is generated based on the X-rays detected in one line. Meanwhile, the X-ray detection camera 10 may have a configuration of a single-line X-ray camera in which one set of configurations for detecting X-rays is disposed, a multi-line X-ray camera in which two or more sets of configurations for detecting X-rays are disposed, or two or more single-line X-ray cameras.

The X-ray detection camera 10 includes a filter 19, scintillators 11a and 11b, line scan cameras 12a and 12b, a sensor control unit 13, amplifiers 14a and 14b, AD converters 15a and 15b, correction circuits 16a and 16b, output interfaces 17a and 17b, and an amplifier control unit 18. The scintillator 11a, the line scan camera 12a, the amplifier 14a, the AD converter 15a, the correction circuit 16a, and the output interface 17a are electrically connected to each other, and have components related to the first line. In addition, the scintillator 11b, the line scan camera 12b, the amplifier 14b, the AD converter 15b, the correction circuit 16b, and the output interface 17b are electrically connected to each other, and have components related to the second line. The line scan camera 12a of the first line and the line scan camera 12b of the second line are disposed side by side in the transport direction TD. Meanwhile, hereinafter, the components of the first line will be described to represent components common to the first line and the second line.

The scintillator 11a is fixed on the line scan camera 12a by adhesion or the like, and converts the X-rays passing through the subject F into scintillation light. The scintillator 11a outputs the scintillation light to the line scan camera 12a. The filter 19 transmits a predetermined wavelength region of the X-rays toward the scintillator 11a. The filter 19 is not necessarily required and may be omitted as appropriate.

The line scan camera 12a detects the scintillation light from the scintillator 11a, converts the detected light into electric charge, and outputs it as a detection signal (electrical signal) to the amplifier 14a. The line scan camera 12a has a plurality of line sensors arranged in parallel in a direction intersecting the transport direction TD. The line sensor is, for example, a charge coupled device (CCD) image sensor, a complementary metal-oxide semiconductor (CMOS) image sensor, or the like, and includes a plurality of photodiodes.

The sensor control unit 13 controls the line scan cameras 12a and 12b to repeatedly capture images at a predetermined detection period so that the line scan cameras 12a and 12b can capture an image of X-rays passing through the same region of the subject F. As the predetermined detection period, for example, a period common to the line scan cameras 12a and 12b may be set based on the distance between the line scan cameras 12a and 12b, the speed of the belt conveyor 60, the distance between the X-ray irradiator 50 and the subject F on the belt conveyor 60 (focus object distance (FOD)), and the distance between the X-ray irradiator 50 and the line scan cameras 12a and 12b (focus detector distance (FDD)). In addition, the predetermined period may be individually set based on the pixel width of a photodiode in a direction perpendicular to the arrangement direction of pixels of the line sensors of the line scan cameras 12a and 12b. In this case, the deviation (delay time) of the detection period between the line scan cameras 12a and 12b may be specified in accordance with the distance between the line scan cameras 12a and 12b, the speed of the belt conveyor 60, the distance between the X-ray irradiator 50 and the subject F on the belt conveyor 60 (FOD), and the distance between the X-ray irradiator 50 and the line scan cameras 12a and 12b (FDD), and individual periods may be set for each. The amplifier 14a amplifies the detection signal at a predetermined set amplification factor to generate an amplified signal, and outputs the amplified signal to the AD converter 15a. The set amplification factor is an amplification factor which is set by the amplifier control unit 18. The amplifier control unit 18 sets the set amplification factor of the amplifiers 14a and 14b based on predetermined imaging conditions.

The AD converter 15a converts the amplified signal (voltage signal) output by the amplifier 14a into a digital signal, and outputs the converted signal to the correction circuit 16a. The correction circuit 16a performs a predetermined correction such as signal amplification on the digital signal, and outputs the corrected digital signal to the output interface 17a. The output interface 17a outputs the digital signal to the outside of the X-ray detection camera 10. In FIG. 1, the AD converter, the correction circuit, and the output interface exist individually, but they may be integrated into one.

The control device 20 is a computer such as, for example, a personal computer (PC). The control device 20 generates an X-ray image based on the digital signal (amplified signal) output from the X-ray detection camera 10 (more specifically, the output interfaces 17a and 17b). The control device 20 generates one X-ray image by performing average processing or addition processing on two digital signals output from the output interfaces 17a and 17b. The generated X-ray image is output to the display device 30 after a noise removal process to be described later is performed, and is displayed by the display device 30. In addition, the control device 20 controls the X-ray irradiator 50, the amplifier control unit 18, and the sensor control unit 13. Meanwhile, the control device 20 of the present embodiment is a device which is independently provided outside the X-ray detection camera 10, but it may be integrated inside the X-ray detection camera 10.

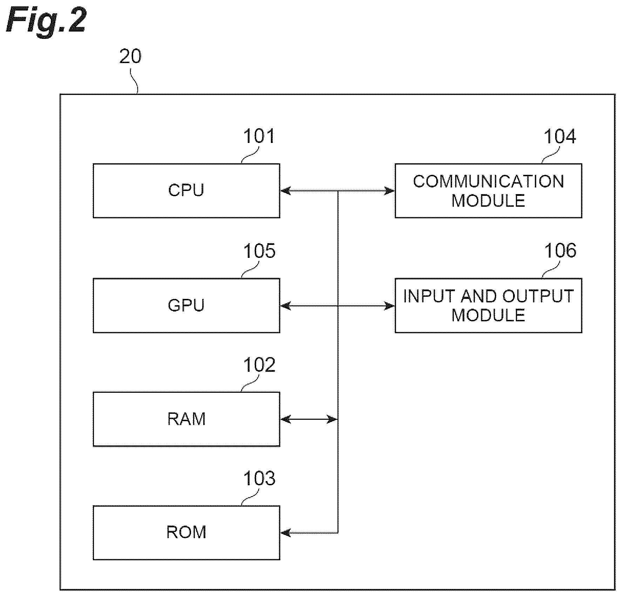

FIG. 2 shows a hardware configuration of the control device 20. As shown in FIG. 2, the control device 20 is a computer or the like physically including a central processing unit (CPU) 101 and graphic processing unit (GPU) 105 which are processors, a random access memory (RAM) 102 and a read only memory (ROM) 103 which are recording media, a communication module 104, an input and output module 106, and the like, which are electrically connected to each other. Meanwhile, the control device 20 may include a display, a keyboard, a mouse, a touch panel display, and the like as the input device 40 and the display device 30, or may include a data recording device such as a hard disk drive and a semiconductor memory. In addition, the control device 20 may be constituted by a plurality of computers.

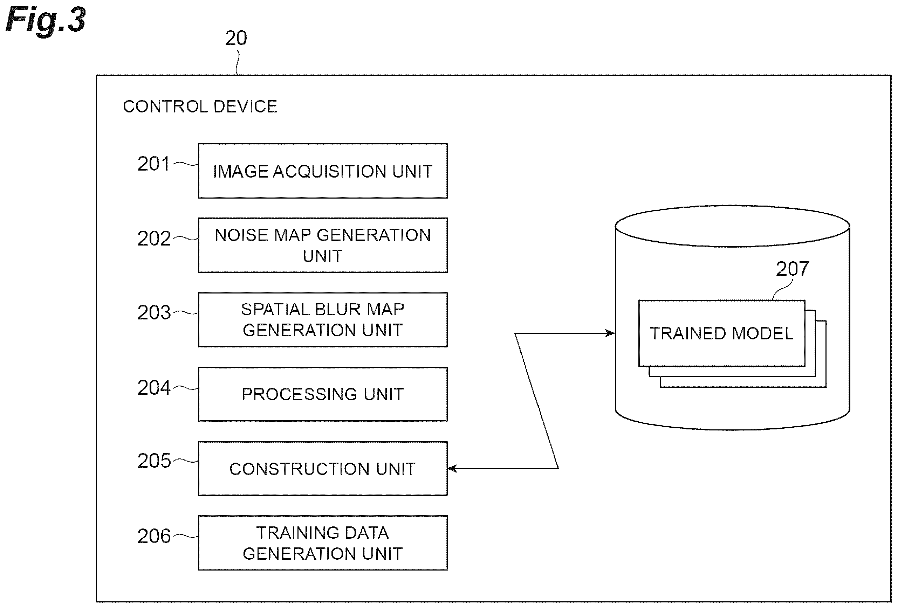

FIG. 3 is a block diagram illustrating a functional configuration of the control device 20. The control device 20 includes an image acquisition unit 201, a noise map generation unit 202, a spatial blur map generation unit 203, a processing unit 204, a construction unit 205, and a training data generation unit 206. Each functional unit of the control device 20 shown in FIG. 3 is realized by loading a program (a radiological image processing program of the present embodiment) on the hardware such as the CPU 101, the GPU 105, and the RAM 102 to thereby bring the communication module 104, the input and output module 106, and the like into operation under the control of the CPU 101 and the GPU 105 and read out and write data in the RAM 102. The CPU 101 and the GPU 105 of the control device 20 cause the control device 20 to function as each functional unit in FIG. 3 by executing this computer program, and sequentially execute processing corresponding to an image processing method to be described later. Meanwhile, the CPU 101 and the GPU 105 may be a single piece of hardware, or only one may be used. In addition, the CPU 101 and the GPU 105 may be implemented in a programmable logic such as an FPGA like a soft processor. The RAM or the ROM may also be a single piece of hardware, or may be built into a programmable logic such as an FPGA. Various types of data required for executing this computer program and various types of data generated by executing this computer program are all stored in a built-in memory such as the ROM 103 or the RAM 102, or a storage medium such as a hard disk drive. In addition, a built-in memory or a storage medium in the control device 20 stores in advance a trained model 207 which is read by the CPU 101 and the GPU 105 and causes the CPU 101 and the GPU 105 to execute noise removal processing on an image (which will be described later).

The details of the function of each functional unit of the control device 20 will be described below.

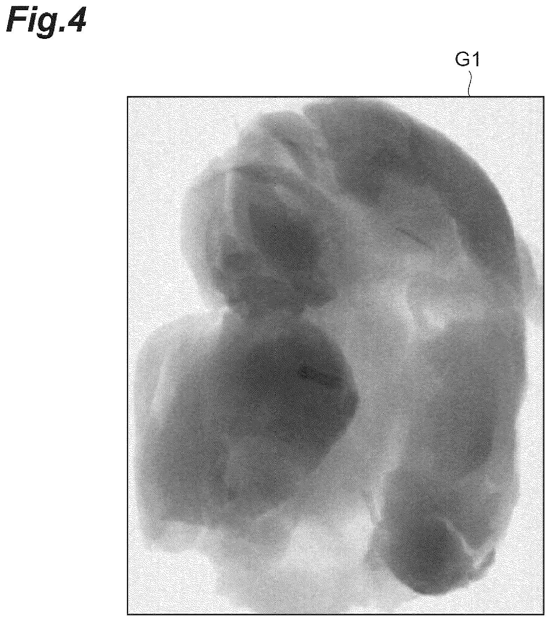

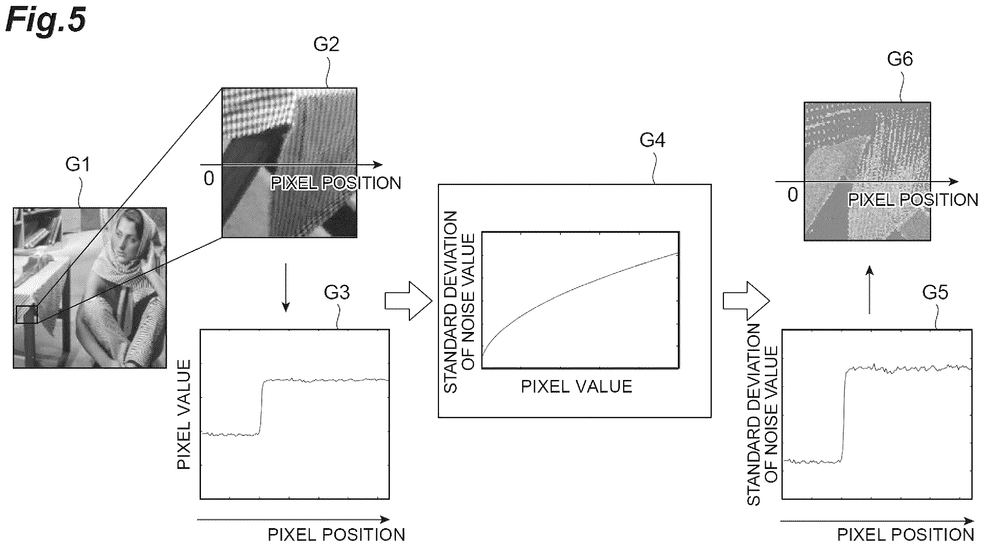

The image acquisition unit 201 acquires an image obtained by irradiating the subject F with an energy beam and capturing an image of the energy beam transmitted through the subject F. Specifically, the image acquisition unit 201 generates an X-ray image based on the digital signal (amplified signal) output from the X-ray detection camera 10 (more specifically, the output interfaces 17a and 17b). The image acquisition unit 201 generates one X-ray image by performing average processing or addition processing on two digital signals output from the output interfaces 17a and 17b. FIG. 4 is a diagram illustrating an example of an X-ray image acquired by the image acquisition unit 201. The image acquisition unit 201 cuts out an image into a plurality of partial images (a plurality of first partial images). For example, as shown in FIG. 5, the image acquisition unit 201 divides an image (radiological image) G1 into a plurality of partial images G2.

The noise map generation unit 202 derives a noise evaluation value from the pixel value of each pixel in the image based on first relationship data indicating a relationship between the pixel value and the noise evaluation value obtained by evaluating the spread of the noise value. The noise map generation unit 202 generates a noise map that is data in which the derived noise evaluation value is associated with each pixel in the image. Specifically, the noise map generation unit 202 acquires a plurality of partial images generated by the image acquisition unit 201. The noise map generation unit 202 derives the standard deviation of pixel values from the pixel value of each pixel in the partial image based on the first relationship data. The noise map generation unit 202 generates a noise map that is data in which the standard deviation of the derived pixel values is associated with each pixel in the partial image. In this case, the noise map generation unit 202 derives a noise evaluation value from the average energy related to the energy beam transmitted through the subject F and the pixel value of each pixel in the partial image.

First, the noise map generation unit 202 calculates the average energy related to the energy beam transmitted through the subject F. As an example, the noise map generation unit 202 calculates the average energy related to the X-rays (radiation) transmitted through the subject F based on condition information input by the input device 40 or the like. The condition information is either the conditions of the source of the energy beam or the imaging conditions when the energy beam is radiated to capture an image of the subject F. Meanwhile, the noise map generation unit 202 may accept an input of the condition information as a direct input of information such as numerical values, or may accept the input as a selective input for information such as numerical values which are set in an internal memory in advance. The noise map generation unit 202 accepts the input of the above condition information from a user, but it may acquire some condition information (such as a tube voltage) in accordance with the detection result of the state of control performed by the control device 20.

The condition information is, for example, information indicating the operating conditions of the X-ray irradiator (source) 50 when the X-ray image of the subject F is captured, the imaging conditions of the X-ray detection camera 10, or the like. Examples of the operating conditions include all or some of the type of X-ray source, a tube voltage, a tube current, a target angle, a target material, and the like. Examples of the condition information indicating the imaging conditions include all or some of the following: the material, thickness, and density of the filters 51 and 19 disposed between the X-ray irradiator 50 and the X-ray detection camera 10, the distance (FDD) between the X-ray irradiator 50 and the X-ray detection camera 10, the type and thickness of window material of the X-ray detection camera 10, information relating to the scintillators 11a and 11b of the X-ray detection camera 10 (for example, thickness, material, density, multiplication factor, surface reflectance, diffusion coefficient, or absorption coefficient), X-ray detection camera information (for example, a gain setting value, a circuit noise value, an amount of saturated charge, a conversion coefficient value (e-/count), or the line rate (Hz) or line speed (m/min) of the camera), information on the subject F (measured substance, thickness, or density), and the like.

For example, the noise map generation unit 202 calculates the spectrum of X-rays detected by the X-ray detection camera 10 using, for example, a known Tucker approximation or the like based on information included in the condition information, such as a tube voltage, a target angle, a target material, the material and thickness of the filters 51 and 19, the presence or absence of the filters 51 and 19, the type of window material of the X-ray detection camera 10 and its presence or absence, and the material and thickness of the scintillators 11a and 11b of the X-ray detection camera 10. The noise map generation unit 202 further calculates a spectral intensity integration value and a photon number integration value from the spectrum of the X-rays, and calculates the value of the average energy of the X-rays by dividing the spectral intensity integration value by the photon number integration value. Meanwhile, for the calculation of the X-ray spectrum, a known Kramers or Birch approximation or the like may be used.

Next, the noise map generation unit 202 uses the first relationship data indicating the relationship between pixel values and the standard deviation of the pixel value (noise evaluation value obtained by evaluating the spread of the noise value) to derive the standard deviation of the pixel values from the calculated average energy of the energy beam and the pixel value of each pixel in the partial image. The noise map generation unit 202 generates a noise map by associating the derived standard deviation of the pixel values with each pixel in the partial image.

For example, the noise map generation unit 202 derives, through a simulation, the first relationship data indicating the relationship between the pixel value and the noise evaluation value obtained by evaluating the spread of the noise value. The relational expression between the pixel values, the average energy of the X-rays, and the standard deviation of the pixel values used by the noise map generation unit 202 is expressed by the following Formulas (1) to (4).

*Noise*

$$= \sqrt{ \frac{ \left( FE_m MCQ \sqrt{\frac{cf}{E_m MCQ + E_m M_{Si} rate_{si}}} \cdot (Signal * C_s - offset) \right)^2 + \left( E_m M_{Si} \sqrt{rate_{si} \frac{cf}{E_m MCQ + E_m M_{Si} rate_{si}}} \cdot (Signal * C_s - offset) \right)^2 + (R)^2 }{\sqrt{N}} } \Bigg/ cf/coeff_{noise} \quad (1)$$

$$F = \sqrt{F_p{}^2 + \frac{1}{MCQ}} \quad\quad\quad (2)$$

$$M = M coeff_M \quad\quad\quad (3)$$

$$F_p = F_p coeff_{Fp} \quad\quad\quad (4)$$

In Formulas (1) to (3), the variable Noise is information indicating the standard deviation of the pixel values, the variable Signal is information indicating the signal value (pixel value) of a pixel, the constant F is information indicating the noise factor, the variable $E_m$ is information indicating the average energy of the X-rays, the constant M is information indicating the multiplication factor by the scintillator, the constant $coeff_M$ is information indicating a coefficient for adjusting the multiplication factor by the scintillator, the constant C is information indicating the coupling efficiency between the line scan camera 12a and the scintillator 11a, or between the line scan camera 12b and the scintillator 11b in the X-ray detection camera 10, and the constant Q is information indicating the quantum efficiency of the line scan camera 12a or the line scan camera 12b.

In Formula (1), the constant cf is information indicating a conversion coefficient for converting the signal value of a pixel into an electric charge in the line scan camera 12a or the line scan camera 12b, and the constant R is information indicating readout noise in the line scan camera 12a or the line scan camera 12b. The conversion coefficient cf and the readout noise R are determined by the gain settings in the line scan camera 12a or the line scan camera 12b.

In Formula (1), the constant $M_{Si}$ is information indicating the multiplication factor of a line scan camera (silicon) when X-rays incident on the scintillator 11a or the scintillator 11b are incident on the line scan camera 12a or the line scan camera 12b without being converted into visible light, the constant $rate_{si}$ is information indicating the silicon direct occurrence rate indicating the probability that X-rays incident on the scintillator 11a or the scintillator 11b are incident on the line scan camera 12a or the line scan camera 12b without being converted into visible light, the constant $C_S$ is information indicating a shading correction value, and the constant offset is information indicating a camera offset indicating the offset value of the line scan cameras 12a and 12b. In Formula (1), the constant N is information indicating the number of sensors. The constant N may be, for example, information indicating the number of line scan cameras (the number of lines), or may be information indicating the binning setting in the line scan camera 12a or the line scan camera 12b. In Formula (1), $coeff_{noise}$ is a coefficient for adjusting noise. In Formulas (2) and (4), the constant $F_p$ is information indicating a coefficient indicating blur, and the constant $coeff_{Fp}$ is information indicating a coefficient for adjusting the coefficient indicating blur.

When Formulas (1) to (4) are used, the noise map generation unit 202 substitutes the pixel value of each pixel in the X-ray image acquired by the image acquisition unit 201 into the variable Signal, and substitutes the numerical value of the average energy calculated by the noise map generation unit 202 into the variable $E_m$. The noise map generation unit 202 obtains the variable Noise calculated using Formulas (1) to (4) as the numerical value of the standard deviation of pixel values. Meanwhile, other parameters including the average energy may be acquired by the noise map generation unit 202 accepting an input, or may be set in advance.

In addition, for example, the noise map generation unit 202 may derive the first relationship data indicating the relationship between the pixel values and the standard deviation of the pixel values based on an image obtained by actual image capturing. As an example, the noise map generation unit 202 may acquire an X-ray image captured by irradiating a jig with X-rays. As the jig, a flat plate-like member or the like of which the thickness and material are known is used. The noise map generation unit 202 may derive relationship data indicating the relationship between the pixel values and the standard deviation of the pixel values from the acquired X-ray image.

The derivation of the first relationship data indicating the relationship between the pixel values and the standard deviation of the pixel values from the X-ray image obtained by capturing an image of the jig which is performed by the noise map generation unit 202 will be described. For the jig, for example, a member of which the thickness changes stepwise in one direction can be used. First, in the X-ray image obtained by capturing an image of the jig, the noise map generation unit 202 derives a pixel value in a case where there is no noise for each step of the jig (hereinafter referred to as a true pixel value), and derives the standard deviation of the pixel values based on the true pixel value. Specifically, the noise map generation unit 202 derives the average value of the pixel values at a certain step of the jig. The noise map generation unit 202 then uses the derived average value of the pixel values as the true pixel value at that step. In that step, the noise map generation unit 202 derives the difference between each pixel value and the true pixel value as a noise value. The noise map generation unit 202 derives the standard deviation of the pixel values from the derived noise value for each pixel value.

The noise map generation unit 202 then derives the first relationship data based on the relationship between the true pixel value and standard deviation of the pixel values. Specifically, the noise map generation unit 202 derives the true pixel value and the standard deviation of the pixel values for each step of the jig. The noise map generation unit 202 plots the relationship between the derived true pixel value and the standard deviation of the pixel values on a graph and draws an approximation curve to derive a relationship graph indicating the relationship between the pixel values and the standard deviation of the pixel values. Meanwhile, for the approximation curve, exponential approximation, linear approximation, log approximation, polynomial approximation, power approximation, and the like are used.

FIG. 5 is a diagram illustrating an example of generation of a noise map which is performed by the noise map generation unit 202. The noise map generation unit 202 derives a relationship graph G4 (first relationship data) indicating a correspondence relation between pixel values and the standard deviation of the pixel values in the captured image of an energy beam. The noise map generation unit 202 acquires a plurality of partial images G2 generated by the image acquisition unit 201. The noise map generation unit 202 then derives relationship data G3 indicating the correspondence relation between each pixel position and the pixel value from the partial image G2. Further, the noise map generation unit 202 derives the standard deviation of the pixel values corresponding to the pixel at each pixel position in the partial image by applying the correspondence relation indicated by the relationship graph G4 to each pixel value in the relationship data G3. As a result, the noise map generation unit 202 associates the derived standard deviation of the pixel values with each pixel position, and derives relationship data G5 indicating the correspondence relation between each pixel position and the standard deviation of the pixel values. The noise map generation unit 202 then generates a noise map G6 based on the derived relationship data G5.

The spatial blur map generation unit 203 generates a spatial blur map indicating the distribution of spatial blur of noise based on the image. Specifically, the spatial blur map generation unit 203 generates a spatial blur map for each of a plurality of partial images.

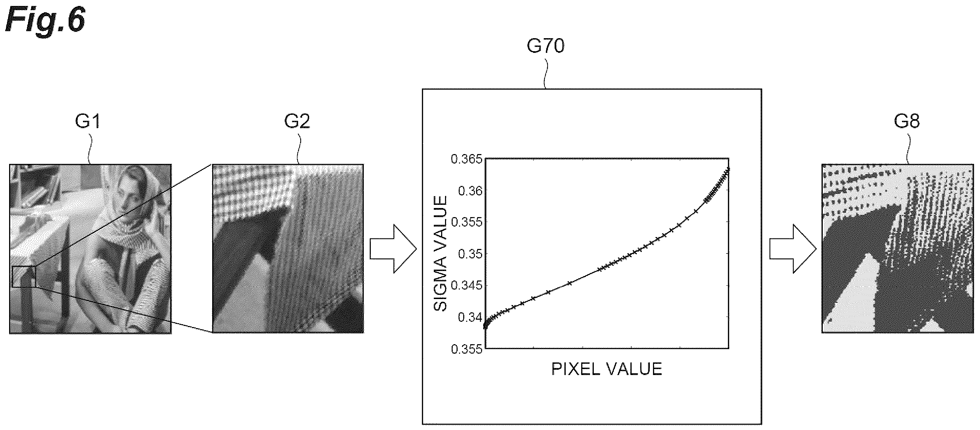

More specifically, first, the spatial blur map generation unit 203 acquires a plurality of partial images generated by the image acquisition unit 201. FIG. 6 is a diagram illustrating an example of generation of a spatial blur map which is performed by the spatial blur map generation unit 203. In the example shown in FIG. 6, the spatial blur map generation unit 203 acquires the partial image G2 generated by the image acquisition unit 201 by cutting out the image G1.

Next, the spatial blur map generation unit 203 derives blur evaluation information from the pixel value of each pixel in the partial image based on second relationship data indicating the relationship between the pixel values and the blur evaluation information obtained by evaluating the spatial blur of noise. In the example shown in FIG. 6, the spatial blur map generation unit 203 derives second relationship data G70 indicating the correspondence relation between pixel values and sigma values in the captured image of the energy beam. The spatial blur map generation unit 203 derives blur evaluation information corresponding to the pixel at each pixel position in the partial image G2 by applying the correspondence relation indicated by the second relationship data G70 to each pixel value in the partial image G2.

The blur evaluation information is an index for evaluating the spatial blur of noise (magnitude of noise blur), for example, a sigma value. The sigma value is an index indicating the spatial spread in a Gaussian distribution or the like. The blur evaluation information may be, for example, at least one of a half width, a point spread function (PSF), a contrast transfer function (CTF), and a modulation transfer function (MTF). Meanwhile, in a case where the blur evaluation information is a contrast transfer function or a modulation transfer function, it has a plurality of parameters. In this case, the spatial blur map is using a plurality of channels.

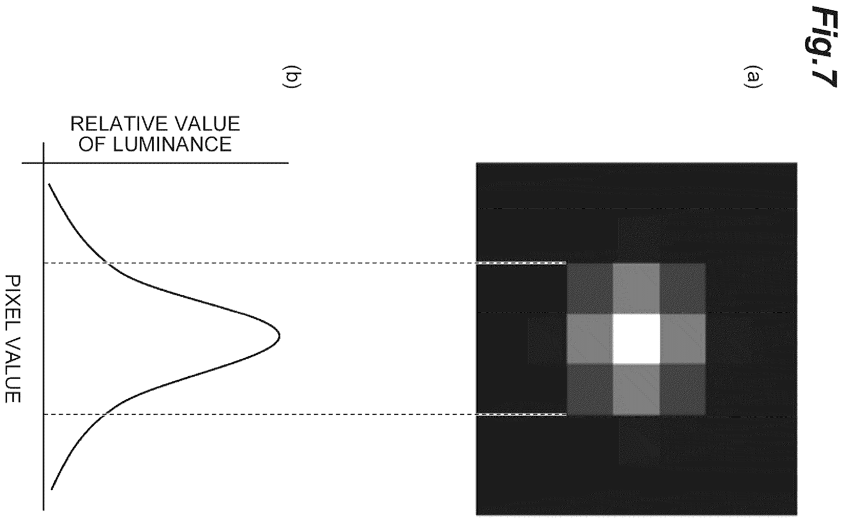

FIGS. 7(a) and 7(b) are diagrams illustrating the spread (spatial spread) of luminance from a predetermined pixel. In FIG. 7(b), the vertical axis represents the relative value of luminance, and the horizontal axis represents the pixel value. As shown in FIG. 7, the spatial blur of noise refers to the spread of luminance (pixel value) at a predetermined pixel to the surrounding area of the pixel. The change in the spatial blur of noise refers to a change in the spread of luminance (frequency of noise). For example, when the spread of luminance in FIG. 7(a) increases (when the frequency of noise decreases), the half width of the graph in FIG. 7(b) increases. In addition, for example, when the spread of luminance in FIG. 7(a) decreases (when the frequency of noise increases), the half width of the graph in FIG. 7(b) decreases. Meanwhile, the spatial blur of noise varies depending on the operating conditions of the energy beam source, the type and thickness of the scintillator, and the like.

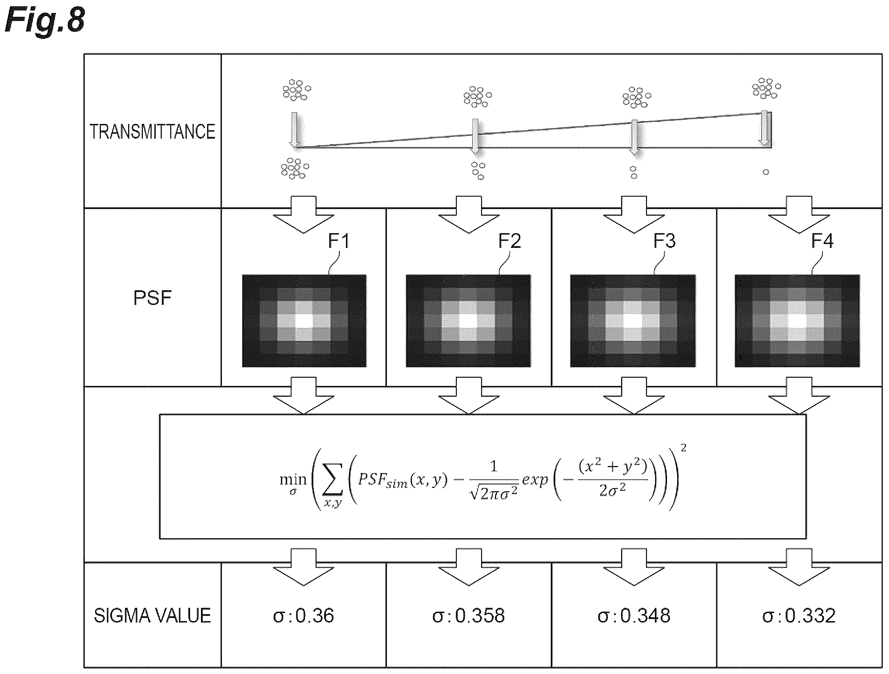

The spatial blur map generation unit 203 generates, through a simulation, the second relationship data indicating the relationship between the pixel value and blur evaluation information obtained by evaluating the spatial blur of noise. Specifically, first, the spatial blur map generation unit 203 acquires condition information. Next, as shown in FIG. 8, the spatial blur map generation unit 203 generates a point spread function (PSF) for each transmittance in the X-ray image by executing a Monte Carlo simulation or the like based on the parameters included in the condition information. Here, the transmittance in the X-ray image may be, for example, a value obtained by dividing the pixel value of each pixel by the background luminance, or may be a value obtained by dividing the pixel value of each pixel by a pixel value set in advance. As shown in the following Formula (5), the spatial blur map generation unit 203 derives the sigma value σ as blur evaluation information for each point spread function by approximating the generated point spread function with a Gaussian function. Finally, the spatial blur map generation unit 203 derives the correspondence relation between the transmittance and the sigma value, and generates the second relationship data indicating the relationship between the pixel value and the sigma value by multiplying the transmittance by the background luminance or a pixel value set in advance.

[Math. 2]

$$\min_{\sigma} \left( \sum_{x,y} \left( PSF_{sim}(x,y) - \frac{1}{\sqrt{2\pi\sigma^2}} exp\left(-\frac{(x^2+y^2)}{2\sigma^2}\right)\right)\right)^2 \quad (5)$$

In Formula (5), the variable σ is information indicating the sigma value, the function $PSF_{sim}$ is information indicating a point spread function generated through a simulation, the variable x is information indicating the x coordinate in the point spread function and the Gaussian function, and the variable y is information indicating the y coordinate in the point spread function and the Gaussian function.

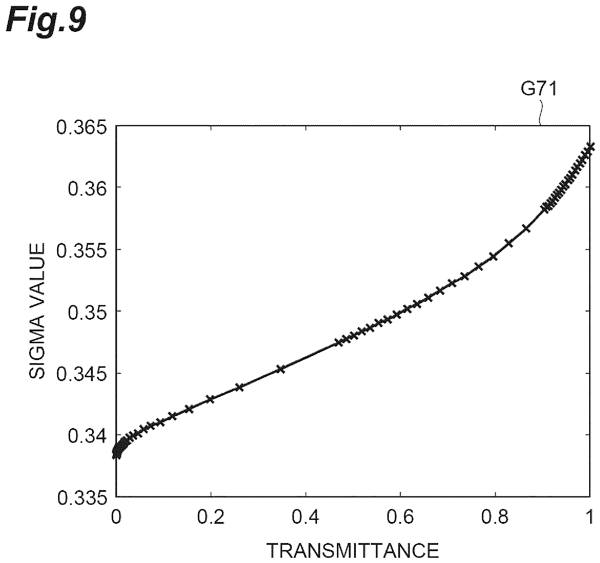

In the example shown in FIG. 8, the spatial blur map generation unit 203 generates point spread functions F1 to F4 for each transmittance in the X-ray image by executing a Monte Carlo simulation based on the condition information. By approximating the point spread functions F1 to F4 with a Gaussian function, the spatial blur map generation unit 203 derives the sigma values σ to be 0.36, 0.358, 0.348, and 0.322, respectively. By associating the derived sigma value with the transmittance, the spatial blur map generation unit 203 derives a correspondence relation G71 between the transmittance and the sigma value shown in FIG. 9. In FIG. 9, the horizontal axis represents the transmittance, and the vertical axis represents the sigma value. Finally, the second relationship data G70 indicating the relationship between the pixel value and the sigma value shown in FIG. 6 is derived by multiplying the transmittance of the above correspondence relation G71 by the background luminance or a luminance set in advance.

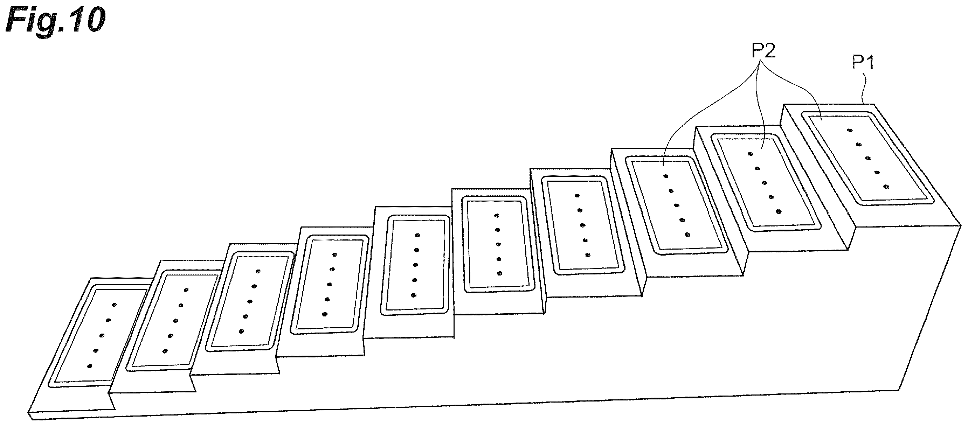

The spatial blur map generation unit 203 may generate the second relationship data indicating the relationship between the pixel value and blur evaluation information obtained by evaluating the spatial blur of noise based on an image obtained by actual image capturing. Specifically, the spatial blur map generation unit 203 may generate the second relationship data based on an image obtained by actually capturing an image of the jig. FIG. 10 shows an example of the structure of the jig. For the jig, for example, a jig P1 that is a member of which the thickness changes stepwise in one direction can be used. A resolution chart P2 is provided at each step of the jig P1.

For example, the spatial blur map generation unit 203 acquires an image obtained by capturing an image of an energy beam transmitted through the jig P1 and the resolution chart P2. The spatial blur map generation unit 203 derives a modulation transfer function (MTF) or a contrast transfer function (CTF) for step of the jig P1 based on the resolution chart P2 shown in the acquired image. The spatial blur map generation unit 203 derives a point spread function (PSF) for step of the jig P1 from the derived modulation transfer function or contrast transfer function. As shown in Formula (5), the spatial blur map generation unit 203 derives the sigma value σ for step of the jig P1 by approximating the generated point spread function with a Gaussian function. The spatial blur map generation unit 203 derives the correspondence relation between the transmittance and the sigma value by associating the transmittance derived for step of the jig P1 with the sigma value of each step of the jig P1. Finally, the spatial blur map generation unit 203 generates the second relationship data indicating the relationship between the pixel value and the sigma value by multiplying the transmittance by the background luminance or a pixel value set in advance. Meanwhile, the derivation of the point spread function from the modulation transfer function described above can be realized by various methods and configurations using known techniques.

Finally, the spatial blur map generation unit 203 generates data in which the derived blur evaluation information is associated with each pixel in the partial image as the spatial blur map. In the example shown in FIG. 6, the spatial blur map generation unit 203 associates the derived blur evaluation information with each pixel position in the partial image G2, and generates a spatial blur map G8 indicating the distribution of spatial blur of noise.

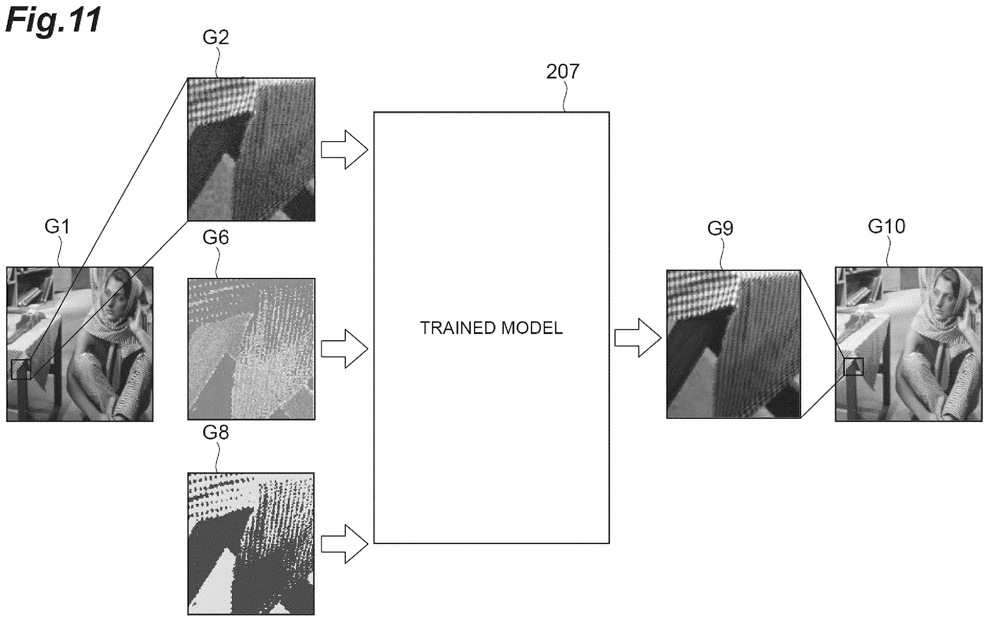

The processing unit 204 inputs the image, the noise map, and the spatial blur map into the trained model 207 constructed in advance through machine learning, and executes image processing for removing noise from the image. Specifically, as shown in FIG. 11, the processing unit 204 acquires the trained model 207 (which will be described later) constructed by the construction unit 205 from a built-in memory or a storage medium within the control device 20. The processing unit 204 inputs the partial image G2 generated by the image acquisition unit 201, the noise map G6 generated by the noise map generation unit 202, and the spatial blur map G8 generated by the spatial blur map generation unit 203 into the trained model 207. The processing unit 204 uses the noise map G6 as a noise weight and uses the spatial blur map G8 as a noise frequency weight. Thereafter, the processing unit 204 uses the trained model 207 to execute image processing for removing noise from each of the plurality of partial images G2, thereby generating a plurality of noise-removed images G9 from which noise has been removed. The processing unit 204 then integrates the plurality of noise-removed images G9 to generate an image G10 from which noise has been removed, and outputs the image G10 to the display device 30 or the like.

The construction unit 205 uses a plurality of partial images (second partial images) generated as training images by cutting out an image (hereinafter referred to as an "entire image"), a noise map and a spatial blur map generated from the partial images, and a noise image in which noise is applied to the partial images, as training data, to construct the trained model 207 through machine learning, the trained model 207 taking the noise map, the spatial blur map, and the noise image as inputs, and outputting a noise-removed image in which noise has been removed from the noise image so that the noise-removed image approximates the partial images. The construction unit 205 stores the constructed trained model 207 in a built-in memory or a storage medium within the control device 20. Examples of machine learning include supervised learning, unsupervised learning, and reinforcement learning, among which are deep learning, neural network learning, and the like. In the present embodiment, the two-dimensional convolutional neural network described in the paper "Beyond a Gaussian Denoiser: Residual Learning of Deep CNN for Image Denoising" authored by Kai Zhang et al. is adopted as an example of a deep learning algorithm. Meanwhile, the trained model 207 may be generated by an external computer or the like and download to the control device 20 in addition to being constructed by the construction unit 205.

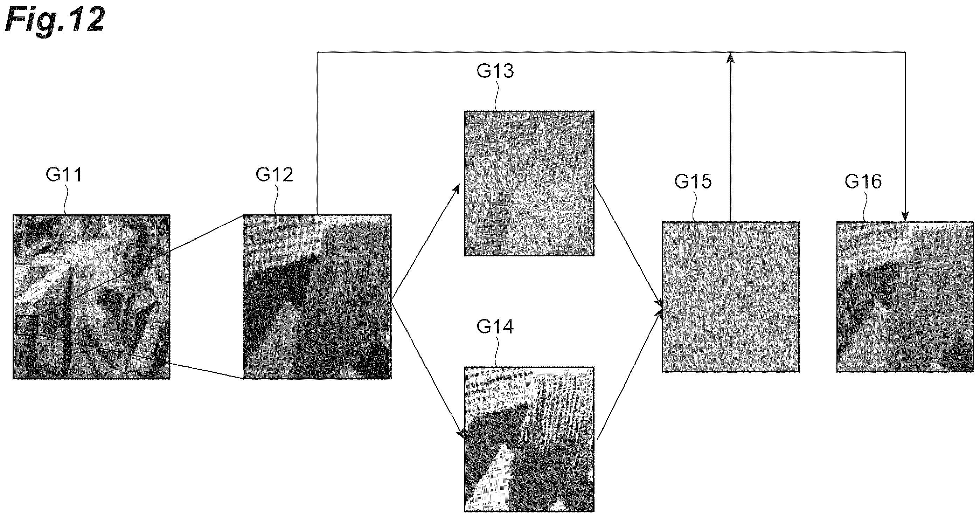

The training data generation unit 206 generates training data used to construct the trained model 207 in the construction unit 205. Specifically, the training data generation unit 206 first acquires an entire image and cuts out a plurality of partial images from the entire image as training images. The entire image used for machine learning may be, for example, an image generated through a simulation, or may be a standard image available through an Internet line or the like. In the example shown in FIG. 12, the training data generation unit 206 cuts out a plurality of partial images G12 from the acquired entire image G11.

Next, the training data generation unit 206 sets a noise evaluation value obtained by evaluating the spread of noise as a noise setting value for each partial image. For the plurality of partial images, the training data generation unit 206 generates a noise map that is data in which the noise evaluation value is associated with each pixel in the partial images based on the noise setting value. In the example shown in FIG. 12, the training data generation unit 206 generates a noise map G13 from the plurality of partial images G12.

Specifically, the training data generation unit 206 sets a different noise evaluation value as a noise setting value for each partial image using any of the following three methods selected. The training data generation unit 206 generates data in which a noise setting value is set in association with each pixel in the partial image as a noise map. In the first method, the training data generation unit 206 sets an arbitrary noise setting value for one partial image. That is, the noise setting value is set uniformly in one partial image, and at the same time, the noise setting value is set randomly in the entire image. In the second method, the training data generation unit 206 further cuts out one partial image into a plurality of regions. The training data generation unit 206 uniformly sets an arbitrary noise setting value for one region. That is, the noise setting value is set randomly in one partial image. Meanwhile, further cutting out (dividing) the partial image into a plurality of regions may be realized, for example, by Otsu's binarization method, or may be realized by various methods and configurations using known techniques. In the third method, in the same way as the noise map generation unit 202, the training data generation unit 206 sets the noise evaluation value derived from the pixel value of each pixel in the partial image as a noise setting value for each pixel in the partial image based on the first relationship data indicating the relationship between the pixel value and the noise evaluation value obtained by evaluating the spread of the noise value. In this case, the training data generation unit 206 may derive the first relationship data through a simulation as described above, or may derive the first relationship data based on an image obtained by actual image capturing (actual measurement).

Next, the training data generation unit 206 sets the blur evaluation information obtained by evaluating the spatial blur of noise as blur setting information for each partial image. The training data generation unit 206 generates a spatial blur map indicating the distribution of spatial blur of noise for the plurality of partial images based on the blur setting information.

Specifically, the training data generation unit 206 sets different blur evaluation information for each partial image as the blur setting information using any of the following three methods selected. The training data generation unit 206 generates data in which the blur setting information is set in association with each pixel in the partial image as the spatial blur map. In the fourth method, the training data generation unit 206 sets arbitrary blur setting information for one partial image. That is, the blur setting information is set uniformly in one partial image, and at the same time, the blur setting information is set randomly in the entire image. In the fifth method, the training data generation unit 206 further cuts out one partial image into a plurality of regions. The training data generation unit 206 uniformly sets arbitrary blur evaluation information for one region. That is, the blur evaluation information is set randomly in one partial image. In the sixth method, in the same way as the spatial blur map generation unit 203, the training data generation unit 206 sets the blur evaluation information as the blur setting information for each pixel in the partial image, the blur evaluation information derived from the pixel value of each pixel in the partial image based on the second relationship data indicating the relationship between the pixel value and the blur evaluation information. In this case, the training data generation unit 206 generates the second relationship data based on a simulation or an image obtained by actual image capturing (actual measurement).

Next, the training data generation unit 206 generates a noise image in which noise is applied to the partial image based on the noise map and the spatial blur map. In the example shown in FIG. 12, the training data generation unit 206 generates noise data G15 based on the noise map G13 and a spatial blur map G14, and generates a noise image G16 by applying the generated noise data G15 to the partial image G12.

The training data generation unit 206 generates a noise distribution having the same size as the partial image based on noise map. Specifically, the training data generation unit 206 determines a noise value for each pixel in the partial image based on the noise map. The training data generation unit 206 generates a noise distribution by associating the determined noise value with each pixel in the partial image.

The training data generation unit 206 applies blur to the above noise distribution based on the spatial blur map to generate noise data. Specifically, the training data generation unit 206 determines in advance the correspondence relation between the blur evaluation information and the point spread function. The training data generation unit 206 determines the point spread function for each pixel by applying the determined correspondence relation to the spatial blur map. The training data generation unit 206 generates noise data by applying blur to the above noise distribution based on the point spread function determined for each pixel. In the example shown in FIG. 12, the training data generation unit 206 generates the noise data G15 by applying blur to the noise distribution based on the spatial blur map G14. Meanwhile, in a case where the blur evaluation information is a point spread function, the training data generation unit 206 generates noise data using the spatial blur map because the point spread function has already been determined for each pixel in the spatial blur map.

More specifically, in a case where the blur evaluation information is a sigma value, the training data generation unit 206 determines in advance the correspondence relation between the sigma value and the point spread function using any of the following three methods selected. In the seventh method, the training data generation unit 206 regards a Gaussian distribution corresponding to the sigma value as a point spread function. In this case, as the Gaussian distribution becomes closer to the point spread function in the actual scintillator, it is possible to construct the trained model 207 in which more effective noise removal can be realized.

In the eighth method, the point spread function is calculated for each scintillator through a simulation in consideration of the type and thickness of the scintillator. The simulation is, for example, a photon Monte Carlo simulation in a scintillator. The training data generation unit 206 determines the sigma value of the Gaussian distribution that most closely approximates the calculated point spread function as the sigma value corresponding to the point spread function. In this case, the correspondence relation between the sigma value and the point spread function can be determined in consideration of a change in the point spread function (light spread function) (blur) due to differences in the type, thickness, and the like of the scintillator. This makes it possible to generate a noise image as training data using a point spread function which is approximated by the blur in an actually captured image. As a result, it is possible to realize more effective noise removal in the trained model 207 constructed using the training data.

In addition, in the eighth method, the training data generation unit 206 may determine the sigma value of the point spread function in further consideration of the X-ray energy spectrum. In this case, the training data generation unit 206 acquires the operating conditions (tube voltage, filter, radiation source information) of the X-ray irradiator 50 included in the condition information, and calculates the energy spectrum of X-rays reaching the scintillators 11a and 11b based on the operating conditions. The training data generation unit 206 determines the sigma value of the point spread function using the above eighth method in consideration of the X-ray energy spectrum. Here, for example, in a case where the X-ray irradiator 50 has a low tube voltage (low energy) even with the same scintillator, a large proportion of light is emitted from the surface side of the scintillator, and the distance that the light travels to the line scan cameras 12a and 12b, resulting in a larger point spread function. In addition, for example, in a case where it has a high tube voltage (high energy) even with the same scintillator, a large proportion of light is emitted from the back side of the scintillator compared to low energy, and the distance that the light travels to the line scan cameras 12a and 12b is short, resulting in a smaller point spread function. In the eighth method, the point spread function is calculated in consideration of the spread of light in the scintillator varying with the X-ray energy. This makes it possible to generate a noise image as training data using a point spread function that more closely approximates the blur in an actually captured image. Thus, it is possible to realize more effective noise removal in the trained model 207 constructed using the training data.

In the ninth method, the training data generation unit 206 acquires an actually measured point spread function. The training data generation unit 206 sets the sigma value of a Gaussian distribution that most closely approximates the acquired point spread function as the sigma value corresponding to the point spread function. In this case, point spread function may be measured for each case where the type and thickness of the scintillator are different, or for each case where the X-ray energy is different.

As shown in FIG. 12, the training data generation unit 206 generates the noise image G16 in which noise is applied to the partial image G12 based on the generated noise data.

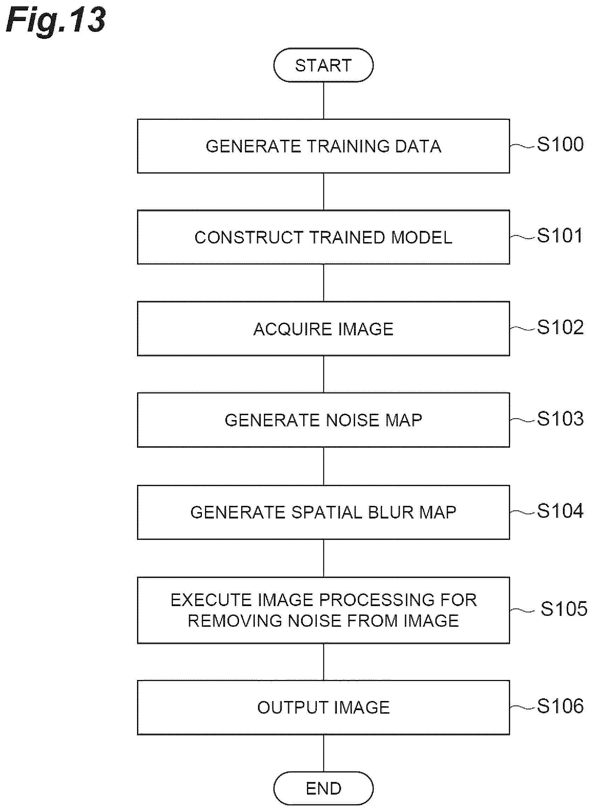

Next, a procedure of observation processing of an energy beam transmission image of the subject F using the image acquisition device 1 according to the present embodiment, that is, a flow of the training method and image processing method according to the present embodiment will be described. FIG. 13 is a flowchart illustrating a procedure of observation processing performed by the image acquisition device 1. Hereinafter, steps S100 and S101 correspond to the training method in the present embodiment. Steps S102 to S106 correspond to the image processing method in the present embodiment.

First, the control device 20 generates training data (step S100: training data generation step). Specifically, the control device 20 first acquires an entire image and cuts out the entire image into a plurality of partial images.

Next, the control device 20 sets a noise evaluation value obtained by evaluating the spread of noise as a noise setting value for each partial image. In this case, the control device 20 sets a different noise evaluation value for each partial image as the noise setting value. For example, the control device 20 uses the above third method to set a noise evaluation value derived from the pixel value of each pixel in the partial image as the noise setting value for each pixel in the partial image based on the first relationship data indicating the relationship between the pixel value and the noise evaluation value obtained by evaluating the spread of the noise value. Additionally, the control device 20 generates a noise map that is data in which the noise evaluation value is associated with each pixel in the image based on the noise setting value for a plurality of partial images. Specifically, the control device 20 generates a noise map which is data in which the noise evaluation value is set in association with each pixel in the partial image.

Next, the control device 20 sets blur evaluation information obtained by evaluating the spatial blur of noise as blur setting information for each partial image. In this case, the control device 20 sets different blur evaluation information for each partial image as the blur setting information. For example, the control device 20 uses the above sixth method to set blur evaluation information as the blur setting information for the pixel in each partial image, the blur evaluation information derived from the pixel value of each pixel in the partial image based on the second relationship data indicating the relationship between the pixel value and the blur evaluation information obtained by evaluating the spatial blur of noise. Additionally, the control device 20 generates a spatial blur map indicating the distribution of spatial blur of noise based on the blur setting information for a plurality of partial images. Specifically, the control device 20 generates data in which the blur setting information is set in association with each pixel in the partial image as the spatial blur map.

Finally, the control device 20 generates a noise image in which noise is applied to the partial image based on the generated noise map and spatial blur map. In this manner, the control device 20 generates a noise map, a spatial blur map, and a noise image.

Next, the control device 20 constructs the trained model 207 (step S101: construction step). Specifically, the control device 20 uses the partial image, the spatial blur map, and the noise image as training data to construct the trained model 207 through machine learning, the trained model 207 taking the spatial blur map and the noise image as inputs, and outputting a noise-removed image in which noise has been removed from the noise image so that the noise-removed image approximates the partial image.

Next, the image acquisition device 1 sets the subject F and captures an image of the subject F, and the control device 20 acquires the image of the subject F (step S102: image acquisition step). Subsequently, the control device 20 generates a noise map (step S103: noise map generation step). Specifically, the control device 20 cuts out the acquired image into a plurality of partial images. The control device 20 derives a noise evaluation value from the pixel value of each pixel in the partial image based on the first relationship data indicating the relationship between the pixel value and the noise evaluation value obtained by evaluating the spread of the noise value, and generates a noise map that is data in which the derived noise evaluation value is associated with each pixel in the partial image.

Next, the control device 20 generates a spatial blur map indicating the distribution of spatial blur of noise based on the acquired image (step S104: spatial blur map generation step). Specifically, the control device 20 derives blur evaluation information from the pixel value of each pixel in the partial image based on the second relationship data indicating the relationship between the pixel value and the blur evaluation information obtained by evaluating the spatial blur of noise, and generates data in which the derived blur evaluation information is associated with each pixel in the partial image as the spatial blur map.

Next, the control device 20 executes image processing for removing noise from the image (step S105: processing step). Specifically, the control device 20 inputs the partial image, the noise map, and the spatial blur map into the trained model 207, and executes image processing for removing noise from the partial image. The plurality of partial images in which noise has been removed are integrated to generate a noise-removed image.

Finally, the processing unit 204 outputs an output image which is an image that has undergone noise removal processing to the display device 30 (step S106).

According to the image acquisition device 1 described above, the spatial blur map indicating the distribution of spatial blur of noise is generated based on the captured image of an energy beam transmitted through the subject F, the image and the spatial blur map are input into the trained model 207 constructed in advance through machine learning, and image processing for removing noise from the image is executed. According to such a configuration, the noise in the image is removed through machine learning in consideration of a change in the blur of noise. For example, even when the measurement conditions and the like of the energy beam change and the blur of noise changes, the noise in the image is effectively removed. This makes it possible to realize noise removal corresponding to a change in the blur of noise in an image using the trained model 207. As a result, it is possible to effectively remove noise in an image.

The above operational effects will be described in detail. In the image acquisition device of the related art, noise in

# EP 4 623 827 A1

an image is removed in consideration of the spread of noise in the luminance direction. For example, noise in an image is removed in consideration of the relationship between the luminance value and the standard deviation of pixel values. However, in a case where the blur of noise which is the spread of noise in a spatial direction changes in the image, a sufficient noise removal effect may not be obtained. For example, in a case where the frequency of noise changes in an X-ray camera due to a difference in the type of scintillator, or the like, it may not be possible to appropriately remove noise. As an example, in image processing methods of the related art, in a case where an image contains high-frequency noise and low-frequency noise, a trained model constructed to remove the high-frequency noise cannot remove the low-frequency noise. In contrast, in the image acquisition device 1 according to the present embodiment, noise is removed from an image in consideration of a change in the frequency of the noise (a change in the blur of the noise). This makes it possible to optimally remove noise even from an image in a situation where the noise has a variety of frequencies. For example, it is possible to effectively remove a plurality of types of noise having different frequencies.

In addition, the control device 20 of the present embodiment derives a noise evaluation value from the pixel value of each pixel in the image based on the first relationship data indicating the relationship between the pixel value and the noise evaluation value obtained by evaluating the spread of the noise value, and generates a noise map that is data in which the derived noise evaluation value is associated with each pixel in the image. The control device 20 inputs the image, the spatial blur map, and the noise map into the trained model 207, and executes image processing for removing noise from the image. According to such a configuration, the spread of the noise value evaluated from the pixel value of each pixel in the image is further considered, and noise in each pixel of the image is removed through machine learning. This makes it possible to use the trained model 207 to realize noise removal that further corresponds to the relationship between the pixel value in the image and the spread of noise. As a result, it is possible to more effectively remove noise in the image.

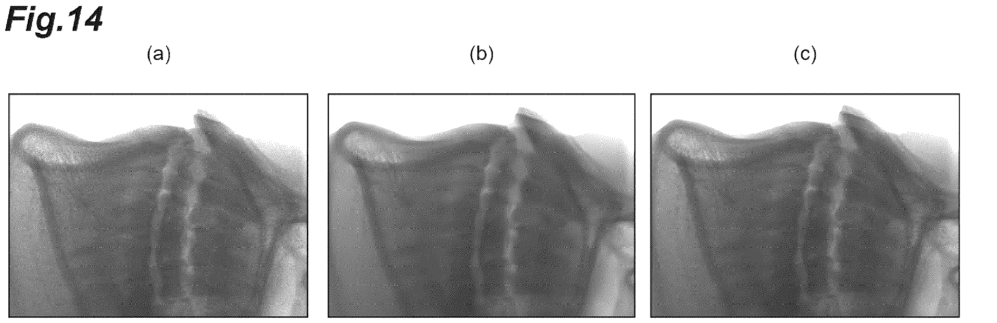

The above operational effects will be described in detail. FIG. 14(a) shows an image before noise is removed. FIG. 14(b) shows an image after noise is removed using an image processing method of the related art. FIG. 14(c) shows an image after noise is removed using the image processing method according to the present embodiment. As shown in FIGS. 14(a) and 14(b), in the image processing method of the related art, in a case where a fine structure of a subject is captured in an image, the fine structure is determined to be noise and removed from the image, which causes the fine structure of the subject to be blurred in the image. In contrast, according to the image processing method of the present embodiment, as shown in FIG. 14(c), the fine structure of the subject is not determined as noise. As a result, noise can be effectively removed without blurring the fine structure in the image, and thus appropriate noise removal can be achieved.

In addition, the control device 20 of the present embodiment generates a spatial blur map for each of a plurality of partial images obtained by cutting out an image, inputs the plurality of partial images into the trained model 207 to execute image processing for removing noise from the plurality of partial images, and integrates the plurality of partial images from which noise has been removed to generate a noise-removed image. According to such a configuration, image processing for removing noise is executed for each of a plurality of partial images obtained by cutting out an image, so that noise in the image can be removed through a simple process.

In addition, the control device 20 of the present embodiment derives blur evaluation information from the pixel value of each pixel in the image based on the second relationship data indicating the relationship between the pixel value and the blur evaluation information obtained by evaluating the spatial blur of noise, and generates data in which the derived blur evaluation information is associated with each pixel in the image as the spatial blur map. According to such a configuration, noise in each pixel of the image is removed through machine learning in consideration of the spatial blur of noise evaluated from the pixel value of each pixel in the image. This makes it possible to more effectively remove noise in the image.

In addition, the control device 20 of the present embodiment cuts out a plurality of partial images from an entire image as training images, sets blur evaluation information obtained by evaluating the spatial blur of noise as blur setting information, and generates a spatial blur map indicating the distribution of spatial blur of noise based on the blur setting information for the plurality of partial images. The control device 20 generates a noise image in which noise is applied to the partial image based on the generated spatial blur map. The control device 20 uses the partial image, the spatial blur map, and the noise image as training data to construct the trained model 207 through machine learning, the trained model 207 taking the spatial blur map and the noise image as inputs, and outputting a noise-removed image in which noise has been removed from the noise image so that the noise-removed image approximates the partial image. According to such a configuration, the trained model 207 used for noise removal in an image is constructed through machine learning using training data. Thereby, when an image and a spatial blur map generated from the image are input into the trained model 207, noise removal corresponding to a change in the spatial blur of noise can be realized. As a result, it is possible to effectively remove noise in an image.

In addition, the control device 20 of the present embodiment sets the blur setting information as different blur evaluation information for each partial image. According to such a configuration, noise removal that makes it possible to cope with various changes in the blur of noise can be realized. As a result, it is possible to more effectively remove noise in the image.

In addition, the control device 20 of the present embodiment sets the blur evaluation information derived from the pixel value of each pixel in the partial image as blur setting information for each pixel in the partial image based on the second relationship data indicating the relationship between the pixel values and the standard deviation of the pixel values, and generates data in which the blur setting information is set in association with each pixel in the partial image as the spatial blur map. According to such a configuration, it is possible to realize noise removal in consideration of the spatial blur of noise evaluated from the pixel value of each pixel in the image. As a result, it is possible to more effectively remove noise in the image.

In addition, the control device 20 of the present embodiment generates the second relationship data through a simulation. According to such a configuration, the second relationship data which is more suitable for noise removal in an image is obtained. As a result, it is possible to more effectively remove noise in the image.

In addition, the control device 20 of the present embodiment generates the second relationship data based on the image obtained by actual image capturing. According to such a configuration, the second relationship data which is more suitable for noise removal in an image is obtained. As a result, it is possible to more effectively remove noise in the image.

In addition, in the control device 20 of the present embodiment, the blur evaluation information is at least one of a sigma value, a half width, a point spread function, a contrast transfer function, and a modulation transfer function. According to such a configuration, noise in an image can be more effectively removed based on information in which the spatial blur of noise is evaluated more specifically.

In addition, in the control device 20 of the present embodiment, the trained model 207 is a model constructed as described above, and causes a processor to execute image processing for removing noise from a captured image of an energy beam transmitted through the subject F. This makes it possible to realize noise removal corresponding to a change in the blur of noise in an image using the trained model 207. As a result, it is possible to effectively remove noise in an image.

Although the embodiment of the present invention has been described above, the present invention is not limited to the above embodiment, and may be modified or applied to other cases without departing from the gist described in the claims.

The processing unit 204 according to the above embodiment inputs a partial image, a spatial blur map, and a noise map into the trained model 207 and executes image processing for removing noise from the partial image, but the noise map does not have to be input into the trained model 207.

For example, the training data generation unit 206 may not generate a noise map as training data. In this case, the training data generation unit 206 may generate a spatial blur map as in the above embodiment, and generate a noise image in which noise is applied to the partial image based on the generated spatial blur map. More specifically, the training data generation unit 206 may determine a noise value for each pixel in the partial image and generate a noise distribution having the same size as the partial image. Each noise value in the noise distribution may be determined based on one standard deviation set in advance, or may be determined arbitrarily using a method other than the above. The training data generation unit 206 may use the partial image, the spatial the blur map, and the noise image as training data to construct the trained model 207 through machine learning, the trained model 207 taking the spatial blur map and the noise image as inputs, and outputting a noise-removed image in which noise has been removed from the noise image so that the noise-removed image approximates the partial image.

In addition, for example, the control device 20 may not include the noise map generation unit 202. In this case, the processing unit 204 may input the partial image and the spatial blur map into the trained model 207 and execute image processing for removing noise from the partial image.

In the flow of the image processing method shown in FIG. 13, in step S100, the control device 20 may generate a noise image in which noise is applied to the partial image based on the generated spatial blur map. In step S101, the control device 20 may use the partial image, the spatial the blur map, and the noise image as training data to construct the trained model 207 through machine learning, the trained model 207 taking the spatial blur map and the noise image as inputs, and outputting a noise-removed image in which noise has been removed from the noise image so that the noise-removed image approximates the partial image. The control device 20 may not execute step S103. In step S105, the control device 20 may input the partial image and the spatial blur map into the trained model 207 and execute image processing for removing noise from the partial image.

The image acquisition unit 201 according to the above embodiment may not cut out an image into a plurality of partial images. For example, the noise map generation unit 202 may generate a noise map for each image. As an example, as shown in FIG. 5, the noise map generation unit 202 may derive the relationship data G3 indicating the correspondence relation between each pixel position and the pixel value from the image G1 instead of the partial image G2. Further, the noise map generation unit 202 may derive the standard deviation of the pixel values corresponding to the pixel at each pixel position in the image G1 by applying the correspondence relation indicated by the relationship graph G4 to each pixel value in the relationship data G3. As a result, the noise map generation unit 202 may associate the derived standard deviation of the pixel values with each pixel position, and derive the relationship data G5 indicating the correspondence relation between each pixel position and the standard deviation of the pixel values. The noise map generation unit 202 may generate the noise map G6 based on the derived relationship data G5.

For example, the spatial blur map generation unit 203 may generate a spatial blur map for each image. As an example, as shown in FIG. 6, the spatial blur map generation unit 203 may derive blur evaluation information corresponding to the pixel at each pixel position in the image G1 by applying the correspondence relation indicated by the second relationship data G70 to each pixel value in the image G1. The spatial blur map generation unit 203 may associate the derived blur evaluation information with each pixel position in the image G1, and generate the spatial blur map G8 indicating the distribution of spatial blur of noise.

For example, the processing unit 204 may input the image, the spatial blur map, and the noise map into the trained model 207 and execute image processing for removing noise from the image. As an example, as shown in FIG. 11, the control device 20 may input the image G1, the noise map G6, and the spatial blur map G8 into the trained model 207 instead of the partial image G2, and execute image processing for removing noise from the image G1. In this case, the control device 20 generates a plurality of noise-removed images G9 from which noise has been removed, and outputs the noise-removed image G9 to the display device 30 or the like.

In the control device 20 according to the above embodiment, the entire image used for machine learning may be an image generated through a simulation, or may be a standard image available through an Internet line or the like, but there is no limitation thereto. For example, the entire image used for machine learning may be a high-output image. In addition, for example, the entire image used for machine learning may be a Noise2Noise image. In this case, the control device 20 may generate a noise map and a sigma map from the Noise2Noise image. The control device 20 may use the Noise2Noise image, the noise map, and the sigma map as training data to construct the trained model 207 through unsupervised learning or the like.

According to the above embodiment, a spatial blur map indicating the distribution of spatial blur of noise is generated based on the captured image of the energy beam transmitted through the subject, the image and the spatial blur map are input into the trained model constructed in advance through machine learning, and image processing for removing noise from the image is executed. According to such a configuration, noise in the image is removed through machine learning in consideration of a change in the blur of the noise. For example, even when the measurement conditions and the like of the energy beam change and the blur of noise changes, the noise in the image is effectively removed. This makes it possible to use a trained model to realize noise removal corresponding to a change in the blur of noise in an image. As a result, it is possible to effectively remove noise in an image.

In addition, it is preferable that the above embodiment further includes a noise map generation step of deriving a noise evaluation value obtained by evaluating a spread of a noise value from a pixel value of each pixel in the image based on first relationship data indicating a relationship between the pixel value and the noise evaluation value, and generating a noise map that is data in which the derived noise evaluation value is associated with each pixel in the image, and the processing step includes inputting the image, the spatial blur map, and the noise map into the trained model and executing image processing for removing noise from the image. In addition, it is preferable that the above embodiment further includes a noise map generation unit configured to derive a noise evaluation value obtained by evaluating a spread of a noise value from a pixel value of each pixel in the radiological image based on first relationship data indicating a relationship between the pixel value and the noise evaluation value, and generate a noise map that is data in which the derived noise evaluation value is associated with each pixel in the radiological image, and the processing unit inputs the radiological image, the spatial blur map, and the noise map into the trained model and executes image processing for removing noise from the radiological image. According to such a configuration, the spread of the noise value evaluated from the pixel value of each pixel in the image is further considered, and noise in each pixel of the image is removed through machine learning. This makes it possible to use the trained model to realize noise removal that further corresponds to the relationship between the pixel value in the image and the spread of noise. As a result, it is possible to more effectively remove noise in the image.

In addition, in the above embodiment, it is preferable that the spatial blur map generation step includes generating the spatial blur map for each of a plurality of first partial images obtained by cutting out the image, and the processing step includes inputting the plurality of first partial images into the trained model instead of the image to execute image processing for removing noise from the plurality of first partial images, and integrating the plurality of first partial images from which noise has been removed to generate the image from which noise has been removed. In addition, in the above embodiment, it is preferable that the spatial blur map generation unit generates the spatial blur map for each of a plurality of first partial images obtained by cutting out the radiological image, and the processing unit inputs the plurality of first partial images into the trained model instead of the radiological image to execute image processing for removing noise from the plurality of first partial images, and integrates the plurality of first partial images from which noise has been removed to generate the radiological image from which noise has been removed. According to such a configuration, image processing for removing noise is executed for each of a plurality of first partial images obtained by cutting out an image, so that noise in the image can be removed through a simple process.

In addition, in the above embodiment, it is preferable that the spatial blur map generation step includes deriving blur evaluation information obtained by evaluating spatial blur of noise from a pixel value of each pixel in the image based on second relationship data indicating a relationship between the pixel value and the blur evaluation information, and generating data in which the derived blur evaluation information is associated with each pixel in the image as the spatial blur map. In addition, in the above embodiment, it is preferable that the spatial blur map generation unit derives blur evaluation information obtained by evaluating spatial blur of noise from a pixel value of each pixel in the radiological image based on second relationship data indicating a relationship between the pixel value and the blur evaluation information, and generates data in which the derived blur evaluation information is associated with each pixel in the radiological image as the spatial blur map. According to such a configuration, noise in each pixel of the image is removed through machine learning in consideration of the spatial blur of noise evaluated from the pixel value of each pixel in the image. This makes it possible to more effectively remove noise in the image.

According to the above embodiment, it is preferable that a training method includes: a training data generation step of cutting out a plurality of second partial images from an entire image as training image, setting blur evaluation information obtained by evaluating spatial blur of noise as blur setting information for each of the second partial images, generating a spatial blur map indicating a distribution of spatial blur of noise for the plurality of second partial images based on the blur setting information, and generating a noise image in which noise is applied to the second partial images based on the generated spatial blur map; and a construction step of using the second partial image, the spatial blur map, and the noise image as training data to construct a trained model through machine learning, the trained model taking the spatial blur map and the noise image as inputs, and outputting a noise-removed image in which noise has been removed from the noise image so that the noise-removed image approximates the second partial image. In addition, it is preferable that the above embodiment further includes: a training data generation unit configured to cut out a plurality of second partial images from an entire image as training image, set blur evaluation information obtained by evaluating spatial blur of noise as blur setting information for each of the second partial images, generate a spatial blur map indicating a distribution of spatial blur of noise for the plurality of second partial images based on the blur setting information, and generate a noise image in which noise is applied to the second partial images based on the generated spatial blur map; and a construction unit configured to use the second partial image, the spatial blur map, and the noise image as training data to construct a trained model through machine learning, the trained model taking the spatial blur map and the noise image as inputs, and outputting a noise-removed image in which noise has been removed from the noise image so that the noise-removed image approximates the second partial image. According to such a configuration, the trained model used for noise removal in an image is constructed through machine learning using training data. Thereby, when an image and a spatial blur map generated from the image are input into the trained model, noise removal corresponding to a change in the spatial blur of noise can be realized. As a result, it is possible to effectively remove noise in an image.

In addition, in the above embodiment, it is preferable that the training data generation step includes setting the blur setting information as the blur evaluation information that differs for each of the second partial images. In addition, in the above embodiment, it is preferable that the training data generation unit sets the blur setting information as the blur evaluation information that differs for each of the second partial images. According to such a configuration, noise removal that makes it possible to cope with various changes in the blur of noise can be realized. As a result, it is possible to more effectively remove noise in the image.

In addition, in the above embodiment, it is preferable that the training data generation step includes setting the blur evaluation information as the blur setting information for each pixel in the second partial image, the blur evaluation information derived from a pixel value of each pixel in the second partial image based on second relationship data indicating a relationship between the pixel value and the blur evaluation information obtained by evaluating spatial blur of noise, and generating data in which the blur setting information is set in association with each pixel in the second partial image as the spatial blur map. In addition, in the above embodiment, the training data generation unit sets the blur evaluation information as the blur setting information for each pixel in the second partial image, the blur evaluation information derived from a pixel value of each pixel in the second partial image based on second relationship data indicating a relationship between the pixel value and the blur evaluation information obtained by evaluating spatial blur of noise, and generates data in which the blur setting information is set in association with each pixel in the second partial image as the spatial blur map. According to such a configuration, it is possible to realize noise removal in consideration of the spatial blur of noise evaluated from the pixel value of each pixel in the image. As a result, it is possible to more effectively remove noise in the image.

In addition, in the above embodiment, it is preferable that the training data generation step includes generating the second relationship data through a simulation. In addition, in the above embodiment, it is preferable that the training data generation unit generates the second relationship data through a simulation. According to such a configuration, the second relationship data which is more suitable for noise removal in an image is obtained. As a result, it is possible to more effectively remove noise in the image.

In addition, in the above embodiment, it is preferable that the training data generation step includes generating the second relationship data based on an image obtained by actual image capturing. In addition, in the above embodiment, it is preferable that the training data generation unit generates the second relationship data based on a radiological image obtained by actual image capturing. According to such a configuration, the second relationship data which is more suitable for noise removal in an image is obtained. As a result, it is possible to more effectively remove noise in the image.

In addition, in the above embodiment, it is preferable that the blur evaluation information is at least one of a sigma value, a half width, a point spread function, a contrast transfer function, and a modulation transfer function. According to such a configuration, noise in an image can be more effectively removed based on information in which the spatial blur of noise is evaluated more specifically.

Alternatively, it is preferable that the trained model of the embodiment is a trained model constructed using the training data generation step and the construction step, including causing a processor to execute image processing for removing noise from an image obtained by capturing an image of an energy beam transmitted through a subject. This makes it possible to use a trained model to realize noise removal corresponding to a change in the blur of noise in an image. As a result, it is possible to effectively remove noise in an image.

The image processing method of the embodiment is [1] "an image processing method comprising: an image acquisition step of acquiring an image obtained by irradiating a subject with an energy beam and capturing an image of the energy beam transmitted through the subject; a spatial blur map generation step of generating a spatial blur map indicating a distribution of spatial blur of noise based on the image; and a processing step of inputting the image and the spatial blur map into a trained model constructed in advance through machine learning and executing image processing for removing noise from the image."

The image processing method of the embodiment may be [2] "the image processing method according to the above [1], further comprising a noise map generation step of deriving a noise evaluation value obtained by evaluating a spread of a noise value from a pixel value of each pixel in the image based on first relationship data indicating a relationship between the pixel value and the noise evaluation value, and generating a noise map that is data in which the derived noise evaluation value is associated with each pixel in the image, wherein the processing step includes inputting the image, the spatial blur map, and the noise map into the trained model and executing image processing for removing noise from the image."

The image processing method of the embodiment may be [3] "the image processing method according to the above [1] or [2], wherein the spatial blur map generation step includes generating the spatial blur map for each of a plurality of first partial images obtained by cutting out the image, and the processing step includes inputting the plurality of first partial images into the trained model instead of the image to execute image processing for removing noise from the plurality of first partial images, and integrating the plurality of first partial images from which noise has been removed to generate the image from which noise has been removed."

The image processing method of the embodiment may be [4] "the image processing method according to any one of the above [1] to [3], wherein the spatial blur map generation step includes deriving blur evaluation information obtained by evaluating spatial blur of noise from a pixel value of each pixel in the image based on second relationship data indicating a relationship between the pixel value and the blur evaluation information, and generating data in which the derived blur evaluation information is associated with each pixel in the image as the spatial blur map."

The training method of the embodiment is [5] "a training method comprising: a training data generation step of cutting out a plurality of second partial images from an entire image as training image, setting blur evaluation information obtained by evaluating spatial blur of noise as blur setting information for each of the second partial images, generating a spatial blur map indicating a distribution of spatial blur of noise for the plurality of second partial images based on the blur setting information, and generating a noise image in which noise is applied to the second partial images based on the generated spatial blur map; and a construction step of using the second partial image, the spatial blur map, and the noise image as training data to construct a trained model through machine learning, the trained model taking the spatial blur map and the noise image as inputs, and outputting a noise-removed image in which noise has been removed from the noise image so that the noise-removed image approximates the second partial image."

The training method of the embodiment may be [6] "the training method according to the above [5], wherein the training data generation step includes setting the blur setting information as the blur evaluation information that differs for each of the second partial images."

The training method of the embodiment may be [7] "the training method according to the above [5] or [6], wherein the training data generation step includes setting the blur evaluation information as the blur setting information for each pixel in the second partial image, the blur evaluation information derived from a pixel value of each pixel in the second partial image based on second relationship data indicating a relationship between the pixel value and the blur evaluation information obtained by evaluating spatial blur of noise, and generating data in which the blur setting information is set in association with each pixel in the second partial image as the spatial blur map."

The training method of the embodiment may be [8] "the training method according to the above [7], wherein the training data generation step includes generating the second relationship data through a simulation."

The training method of the embodiment may be [9] "the training method according to the above [7], wherein the training data generation step includes generating the second relationship data based on an image obtained by actual image capturing."

The training method of the embodiment may be [10] "the training method according to any one of the above [5] to [9], wherein in the training data generation step, the blur evaluation information is at least one of a sigma value, a half width, a point spread function, a contrast transfer function, and a modulation transfer function."

The trained model of the embodiment may be [11] "a trained model constructed using the training method according to any one of the above [5] to [10], comprising causing a processor to execute image processing for removing noise from an image obtained by capturing an image of an energy beam transmitted through a subject."

The radiological image processing module of the embodiment is [12] "a radiological image processing module comprising: an image acquisition unit configured to acquire a radiological image obtained by irradiating a subject with radiation and capturing an image of the radiation transmitted through the subject; a spatial blur map generation unit configured to generate a spatial blur map indicating a distribution of spatial blur of noise based on the radiological image; and a processing unit configured to input the radiological image and the spatial blur map into a trained model constructed in advance through machine learning and execute image processing for removing noise from the radiological image."

The radiological image processing module of the embodiment may be [13] "the radiological image processing module according to the above [12], further comprising a noise map generation unit configured to derive a noise evaluation value obtained by evaluating a spread of a noise value from a pixel value of each pixel in the radiological image based on first relationship data indicating a relationship between the pixel value and the noise evaluation value, and generate a noise map that is data in which the derived noise evaluation value is associated with each pixel in the radiological image, wherein the processing unit configured to input the radiological image, the spatial blur map, and the noise map into the trained model and execute image processing for removing noise from the radiological image."

The radiological image processing module of the embodiment may be [14] "the radiological image processing module according to the above [12] or [13], wherein the spatial blur map generation unit configured to generate the spatial blur map for each of a plurality of first partial images obtained by cutting out the radiological image, and the processing unit configured to input the plurality of first partial images into the trained model instead of the radiological image to execute image processing for removing noise from the plurality of first partial images, and integrate the plurality of first partial images from which noise has been removed to generate the radiological image from which noise has been removed."

The radiological image processing module of the embodiment may be [15] "the radiological image processing module according to any one of the above [12] to [14], wherein the spatial blur map generation unit configured to derive blur evaluation information obtained by evaluating spatial blur of noise from a pixel value of each pixel in the radiological image based on second relationship data indicating a relationship between the pixel value and the blur evaluation information, and generate data in which the derived blur evaluation information is associated with each pixel in the radiological image as the spatial blur map."

The radiological image processing module of the embodiment is [16] "the radiological image processing module according to any one of the above [12] to [15], further comprising: a training data generation unit configured to cut out a plurality of second partial images from an entire image as training image, set blur evaluation information obtained by evaluating spatial blur of noise as blur setting information for each of the second partial images, generate a spatial blur map indicating a distribution of spatial blur of noise for the plurality of second partial images based on the blur setting information, and generate a noise image in which noise is applied to the second partial images based on the generated spatial blur map; and a construction unit configured to use the second partial image, the spatial blur map, and the noise image as training data to construct a trained model through machine learning, the trained model taking the spatial blur map and the noise image as inputs, and outputting a noise-removed image in which noise has been removed from the noise image so that the noise-removed image approximates the second partial image."

The radiological image processing module of the embodiment may be [17] "the radiological image processing module according to the above [16], wherein the training data generation unit configured to set the blur setting information as the blur evaluation information that differs for each of the second partial images."

The radiological image processing module of the embodiment may be [18] "the radiological image processing module according to the above [16] or [17], wherein the training data generation unit configured to set the blur evaluation information as the blur setting information for each pixel in the second partial image, the blur evaluation information derived from a pixel value of each pixel in the second partial image based on second relationship data indicating a relationship between the pixel value and the blur evaluation information obtained by evaluating spatial blur of noise, and generate data in which the blur setting information is set in association with each pixel in the second partial image as the spatial blur map."

The radiological image processing module of the embodiment may be [19] "the radiological image processing module according to the above [18], wherein the training data generation unit configured to generate the second relationship data through a simulation."

The radiological image processing module of the embodiment may be [20] "the radiological image processing module according to the above [18], wherein the training data generation unit configured to generate the second relationship data based on a radiological image obtained by actual image capturing."

The radiological image processing module of the embodiment may be [21] "the radiological image processing module according to any one of the above [16] to [20], wherein the blur evaluation information is at least one of a sigma value, a half width, a point spread function, a contrast transfer function, and a modulation transfer function."

The radiological image processing program of the embodiment may be [22] "a radiological image processing program causing a processor to function as: an image acquisition unit configured to acquire a radiological image obtained by irradiating a subject with radiation and capturing an image of the radiation transmitted through the subject; a spatial blur map generation unit configured to generate a spatial blur map indicating a distribution of spatial blur of noise based on the radiological image; and a processing unit configured to input the radiological image and the spatial blur map into a trained model constructed in advance through machine learning and execute image processing for removing noise from the radiological image."

The radiological image processing system of the embodiment may be [23] "a radiological image processing system comprising: the radiological image processing module according to any one of the above [12] to [21]; a source configured to irradiate the subject with radiation; and an imaging device configured to capture an image of the radiation transmitted through the subject to acquire the radiological image."

**Reference Signs List**

10 X-ray detection camera (imaging device)
20 Control device (radiological image processing module)
50 X-ray irradiator (source)
201 Image acquisition unit
202 Noise map generation unit
203 Spatial blur map generation unit
204 Processing unit
205 Construction unit
206 Training data generation unit
207 Trained model
F Subject
G1, G10 Image (radiological image)
G2 Partial image (first partial image)
G12 Partial image (second partial image)
G4 Relationship graph (first relationship data)
G6, G13 Noise map
G70 Second relationship data
G8, G14 Spatial blur map
G9 Noise-removed image
G11 Entire image
G16 Noise image

**Claims**

1. An image processing method comprising:

   an image acquisition step of acquiring an image obtained by irradiating a subject with an energy beam and capturing an image of the energy beam transmitted through the subject;
   a spatial blur map generation step of generating a spatial blur map indicating a distribution of spatial blur of noise based on the image; and
   a processing step of inputting the image and the spatial blur map into a trained model constructed in advance through machine learning and executing image processing for removing noise from the image.

2. The image processing method according to claim 1, further comprising a noise map generation step of deriving a noise evaluation value obtained by evaluating a spread of a noise value from a pixel value of each pixel in the image based on first relationship data indicating a relationship between the pixel value and the noise evaluation value, and generating a noise map that is data in which the derived noise evaluation value is associated with each pixel in the image, wherein the processing step includes inputting the image, the spatial blur map, and the noise map into the trained model and executing image processing for removing noise from the image.

3. The image processing method according to claim 1 or 2, wherein the spatial blur map generation step includes generating the spatial blur map for each of a plurality of first partial images obtained by cutting out the image, and the processing step includes inputting the plurality of first partial images into the trained model instead of the image to execute image processing for removing noise from the plurality of first partial images, and integrating the plurality of first partial images from which noise has been removed to generate the image from which noise has been removed.

4. The image processing method according to any one of claims 1 to 3, wherein the spatial blur map generation step includes deriving blur evaluation information obtained by evaluating spatial blur of noise from a pixel value of each pixel in the image based on second relationship data indicating a relationship between the pixel value and the blur evaluation information, and generating data in which the derived blur evaluation information is associated with each pixel in the image as the spatial blur map.

5. A training method comprising:

a training data generation step of cutting out a plurality of second partial images from an entire image as training image, setting blur evaluation information obtained by evaluating spatial blur of noise as blur setting information for each of the second partial images, generating a spatial blur map indicating a distribution of spatial blur of noise for the plurality of second partial images based on the blur setting information, and generating a noise image in which noise is applied to the second partial images based on the generated spatial blur map; and
a construction step of using the second partial image, the spatial blur map, and the noise image as training data to construct a trained model through machine learning, the trained model taking the spatial blur map and the noise image as inputs, and outputting a noise-removed image in which noise has been removed from the noise image so that the noise-removed image approximates the second partial image.

6. The training method according to claim 5, wherein the training data generation step includes setting the blur setting information as the blur evaluation information that differs for each of the second partial images.

7. The training method according to claim 5 or 6, wherein the training data generation step includes setting the blur evaluation information as the blur setting information for each pixel in the second partial image, the blur evaluation information derived from a pixel value of each pixel in the second partial image based on second relationship data indicating a relationship between the pixel value and the blur evaluation information obtained by evaluating spatial blur of noise, and generating data in which the blur setting information is set in association with each pixel in the second partial image as the spatial blur map.

8. The training method according to claim 7, wherein the training data generation step includes generating the second relationship data through a simulation.

9. The training method according to claim 7, wherein the training data generation step includes generating the second relationship data based on an image obtained by actual image capturing.

10. The training method according to any one of claims 5 to 9, wherein in the training data generation step, the blur evaluation information is at least one of a sigma value, a half width, a point spread function, a contrast transfer function, and a modulation transfer function.

11. A trained model constructed using the training method according to any one of claims 5 to 10, comprising causing a processor to execute image processing for removing noise from an image obtained by capturing an image of an energy beam transmitted through a subject.

12. A radiological image processing module comprising:

an image acquisition unit configured to acquire a radiological image obtained by irradiating a subject with radiation and capturing an image of the radiation transmitted through the subject;
a spatial blur map generation unit configured to generate a spatial blur map indicating a distribution of spatial blur of noise based on the radiological image; and
a processing unit configured to input the radiological image and the spatial blur map into a trained model constructed in advance through machine learning and execute image processing for removing noise from the radiological image.

13. The radiological image processing module according to claim 12, further comprising a noise map generation unit configured to derive a noise evaluation value obtained by evaluating a spread of a noise value from a pixel value of each pixel in the radiological image based on first relationship data indicating a relationship between the pixel value and the noise evaluation value, and generate a noise map that is data in which the derived noise evaluation value is associated with each pixel in the radiological image,

wherein the processing unit configured to input the radiological image, the spatial blur map, and the noise map into the trained model and execute image processing for removing noise from the radiological image.

14. The radiological image processing module according to claim 12 or 13, wherein the spatial blur map generation unit configured to generate the spatial blur map for each of a plurality of first partial images obtained by cutting out the radiological image, and

the processing unit configured to input the plurality of first partial images into the trained model instead of the radiological image to execute image processing for removing noise from the plurality of first partial images, and integrate the plurality of first partial images from which noise has been removed to generate the radiological image from which noise has been removed.

15. The radiological image processing module according to any one of claims 12 to 14, wherein the spatial blur map generation unit configured to derive blur evaluation information obtained by evaluating spatial blur of noise from a pixel value of each pixel in the radiological image based on second relationship data indicating a relationship between the pixel value and the blur evaluation information, and generate data in which the derived blur evaluation information is associated with each pixel in the radiological image as the spatial blur map.

16. The radiological image processing module according to any one of claims 12 to 15, further comprising:

a training data generation unit configured to cut out a plurality of second partial images from an entire image as training image, set blur evaluation information obtained by evaluating spatial blur of noise as blur setting information for each of the second partial images, generate a spatial blur map indicating a distribution of spatial blur of noise for the plurality of second partial images based on the blur setting information, and generate a noise image in which noise is applied to the second partial images based on the generated spatial blur map; and

a construction unit configured to use the second partial image, the spatial blur map, and the noise image as training data to construct a trained model through machine learning, the trained model taking the spatial blur map and the noise image as inputs, and outputting a noise-removed image in which noise has been removed from the noise image so that the noise-removed image approximates the second partial image.

17. The radiological image processing module according to claim 16, wherein the training data generation unit configured to set the blur setting information as the blur evaluation information that differs for each of the second partial images.

18. The radiological image processing module according to claim 16 or 17, wherein the training data generation unit configured to set the blur evaluation information as the blur setting information for each pixel in the second partial image, the blur evaluation information derived from a pixel value of each pixel in the second partial image based on second relationship data indicating a relationship between the pixel value and the blur evaluation information obtained by evaluating spatial blur of noise, and generate data in which the blur setting information is set in association with each pixel in the second partial image as the spatial blur map.

19. The radiological image processing module according to claim 18, wherein the training data generation unit configured to generate the second relationship data through a simulation.

20. The radiological image processing module according to claim 18, wherein the training data generation unit configured to generate the second relationship data based on a radiological image obtained by actual image capturing.

21. The radiological image processing module according to any one of claims 16 to 20, wherein the blur evaluation information is at least one of a sigma value, a half width, a point spread function, a contrast transfer function, and a modulation transfer function.

22. A radiological image processing program causing a processor to function as:

an image acquisition unit configured to acquire a radiological image obtained by irradiating a subject with radiation and capturing an image of the radiation transmitted through the subject;

a spatial blur map generation unit configured to generate a spatial blur map indicating a distribution of spatial blur of noise based on the radiological image; and

a processing unit configured to input the radiological image and the spatial blur map into a trained model constructed in advance through machine learning and execute image processing for removing noise from the radiological image.

23. A radiological image processing system comprising:

the radiological image processing module according to any one of claims 12 to 21;
a source configured to irradiate the subject with radiation; and
an imaging device configured to capture an image of the radiation transmitted through the subject to acquire the radiological image.

*Fig.1*

Fig. 1 — Block diagram showing components: 1, 10, 20, 30 DISPLAY DEVICE, 40 INPUT DEVICE, CONTROL DEVICE, 50, 51, 60, F, TD, SENSOR CONTROL UNIT 13, AMPLIFIER CONTROL UNIT 18, AMPLIFIER 14a/14b, 15a/15b, CORRECTION CIRCUIT 16a/16b, OUTPUT INTERFACE 17a/17b, 11a/11b, 12a/12b, 19.

*Fig.2*

20

101
CPU

104
COMMUNICATION
MODULE

105
GPU

106
INPUT AND OUTPUT
MODULE

102
RAM

103
ROM

*Fig.3*

CONTROL DEVICE

20

201 — IMAGE ACQUISITION UNIT

202 — NOISE MAP GENERATION UNIT

203 — SPATIAL BLUR MAP GENERATION UNIT

204 — PROCESSING UNIT

205 — CONSTRUCTION UNIT

206 — TRAINING DATA GENERATION UNIT

207

TRAINED MODEL

EP 4 623 827 A1

## Fig.4

G1

Fig.5

# Fig.6

G1

G2

G70

PIXEL VALUE

SIGMA VALUE

G8

# *Fig.7*

(a)

(b)

RELATIVE VALUE
OF LUMINANCE

PIXEL VALUE

**Fig.8**

| | | | | |
|---|---|---|---|---|
| TRANSMITTANCE | | | | |
| PSF | F1 | F2 | F3 | F4 |

$$\min_{\sigma}\left(\sum_{x,y}\left(PSF_{sim}(x,y)-\frac{1}{\sqrt{2\pi\sigma^2}}exp\left(-\frac{(x^2+y^2)}{2\sigma^2}\right)\right)\right)^2$$

| SIGMA VALUE | $\sigma:0.36$ | $\sigma:0.358$ | $\sigma:0.348$ | $\sigma:0.332$ |
|---|---|---|---|---|

EP 4 623 827 A1

Fig.9

Fig.10

Fig.11

EP 4 623 827 A1

Fig.12

G11

G12

G13

G14

G15

G16

EP 4 623 827 A1

## Fig.13

```
            ( START )
                |
   ┌─────────────────────────────┐
   │   GENERATE TRAINING DATA     │── S100
   └─────────────────────────────┘
                |
   ┌─────────────────────────────┐
   │   CONSTRUCT TRAINED MODEL    │── S101
   └─────────────────────────────┘
                |
   ┌─────────────────────────────┐
   │       ACQUIRE IMAGE          │── S102
   └─────────────────────────────┘
                |
   ┌─────────────────────────────┐
   │     GENERATE NOISE MAP       │── S103
   └─────────────────────────────┘
                |
   ┌─────────────────────────────┐
   │  GENERATE SPATIAL BLUR MAP   │── S104
   └─────────────────────────────┘
                |
   ┌─────────────────────────────┐
   │ EXECUTE IMAGE PROCESSING FOR │── S105
   │  REMOVING NOISE FROM IMAGE   │
   └─────────────────────────────┘
                |
   ┌─────────────────────────────┐
   │       OUTPUT IMAGE           │── S106
   └─────────────────────────────┘
                |
             ( END )
```

Fig.14

(a)　(b)　(c)

## INTERNATIONAL SEARCH REPORT

| International application No. |
| --- |
| **PCT/JP2023/033577** |

**A. CLASSIFICATION OF SUBJECT MATTER**

*A61B 6/00*(2024.01)i

FI: A61B6/00 350A; A61B6/00 350Z

According to International Patent Classification (IPC) or to both national classification and IPC

**B. FIELDS SEARCHED**

Minimum documentation searched (classification system followed by classification symbols)

A61B6/00-6/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

PubMed; IEEE Xplore

**C. DOCUMENTS CONSIDERED TO BE RELEVANT**

| Category* | Citation of document, with indication, where appropriate, of the relevant passages | Relevant to claim No. |
| --- | --- | --- |
| A | JP 2019-208990 A (CANON MEDICAL SYSTEMS CORP.) 12 December 2019 (2019-12-12) paragraphs [0088]-[0146], fig. 1-19 | 1-23 |
| A | WO 2022/172506 A1 (HAMAMATSU PHOTONICS K.K.) 18 August 2022 (2022-08-18) entire text, all drawings | 1-23 |
| A | US 2021/0330274 A1 (SIEMENS HEALTHCARE GMBH) 28 October 2021 (2021-10-28) paragraph [0089] | 1-23 |
| A | US 2019/0005686 A1 (SHANGHAI UNITED IMAGING HEALTHCARE CO., LTD.) 03 January 2019 (2019-01-03) paragraph [0130] | 1-23 |
| A | JP 2021-19714 A (HITACHI, LTD.) 18 February 2021 (2021-02-18) entire text, all drawings | 1-23 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **17 November 2023** | **05 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/033577**

| Patent document cited in search report | | | Publication date (day/month/year) | Patent family member(s) | | | Publication date (day/month/year) |
|---|---|---|---|---|---|---|---|
| JP | 2019-208990 | A | 12 December 2019 | US 2019/0378270 A1 paragraphs [0136]-[0207], fig. 1-19 | | | |
| WO | 2022/172506 | A1 | 18 August 2022 | (Family: none) | | | |
| US | 2021/0330274 | A1 | 28 October 2021 | (Family: none) | | | |
| US | 2019/0005686 | A1 | 03 January 2019 | CN | 107292847 | A | |
| JP | 2021-19714 | A | 18 February 2021 | US 2021/0027430 A1 entire text, all drawings CN 112308788 A | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022172506 A

**Non-patent literature cited in the description**

- **KAI ZHANG**. *Beyond a Gaussian Denoiser: Residual Learning of Deep CNN for Image Denoising*